(19) Europäisches Patentamt
European Patent Office
Office européen des brevets

(11) Veröffentlichungsnummer: **0 020 303**
**B1**

(12) # EUROPÄISCHE PATENTSCHRIFT

(45) Veröffentlichungstag der Patentschrift: **28.08.85**

(21) Anmeldenummer: **80810171.1**

(22) Anmeldetag: **23.05.80**

(51) Int. Cl.[4]: **C 07 D 225/02,**
C 07 D 419/12, A 61 K 31/395
// C07D211/16, C07D265/30,
C07D295/20

(54) **Guanidine, Verfahren zur Herstellung und pharmazeutische Präparate enthaltend solche Verbindungen.**

(30) Priorität: **29.05.79 CH 4993/79**

(43) Veröffentlichungstag der Anmeldung:
**10.12.80 Patentblatt 80/25**

(45) Bekanntmachung des Hinweises auf die Patenterteilung:
**28.08.85 Patentblatt 85/35**

(84) Benannte Vertragsstaaten:
**AT BE CH DE FR GB IT LI LU NL SE**

(56) Entgegenhaltungen:
**BE-A- 852 565**
**FR-M- 8 382**
**GB-A-1 409 768**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Postfach**
**CH-4002 Basel (CH)**

(72) Erfinder: **Dave, Krishna G., Dr.**
**CIBA-GEIGY Research Centre Housing Colony**
**Aarey Road Goregaon Bombay 400063 (IN)**
Erfinder: **George, Thomas, Dr.**
**CIBA-GEIGY Research Centre Housing Colony**
**Aarey Road Goregaon Bombay 400063 (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 020 303 B1

**Beschreibung**

Die vorliegende Erfindung betrifft neue durch einen 8-Ringglieder enthaltenden N-Heterocyclus substituierte Guanidinverbindungen.

In der belgischen Patentschrift No. 852 565 werden Guanidine beschrieben, die einen 5-, 6- oder 7-Ringglieder enthaltenden N-heterocyclischen Substituenten aufweisen und hypoglykämisch wirksam sind. Die erfindungsgemässen Guanidine weisen den Vorteil auf, dass sie bei annähernd gleicher hypoglykämischer Wirksamkeit eine kürzere Halbwertzeit und auch keine unerwünschte Glukoneogenesishemmwirkung aufweisen. Kürzere Halbwertzeiten in der hypoglykämischen Wirksamkeit und das Ausbleiben einer Glukoneogenesishemmwirkung gelten heutzutage in der Fachwelt als erstrebenswerte Vorteile.

Die Erfindung betriff neue Guanidinderivate der Formel I

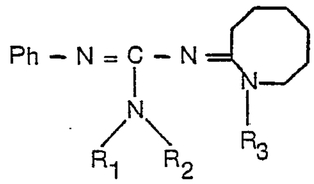

(I)

worin Ph einen gegebenenfalls durch Niederalkyl, Hydroxy, Mercapto, Niederalkoxy, Niederalkenyloxy, Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander Niederalkyl oder Cycloalkyl mit höchstens 10 Kohlenstoffatomen oder beide zusammengenommen einen gegebenenfalls durch Niederalkyl oder Phenyl substituierten bivalenten Kohlenwasserstoffrest aliphatischen Charakters, mit 4—7 Kohlenstoffatomen in der Kette in welchem die Kohlenstoffatome der Kette durch Sauerstoff, Schwefel oder Stickstoff unterbrochen sein können, $R_3$ Wasserstoff oder Niederalkyl bedeuten, ihre tautomeren Verbindungen und Salze.

Im Zusammenhang mit der vorliegenden Beschreibung enthalten die mit "nieder" bezeichneten Reste und Verbindungen vorzugsweise bis zu 7, in erster Linie bis zu 4 Kohlenstoffatome.

Vorstehend wie nachstehend können die Allgemeinbegriffe folgende Bedeutung haben:

Beispielsweise bedeuten $R_1$, $R_2$ und $R_3$ als Niederalkylreste geradkettige oder verzweigte Niederalkylreste und können z.B. Methyl-, Aethyl-, n-Propyl-, Isopropyl-, n-Butyl-, Isobutyl-, sek.-Butyl-, tert.-Butyl-, n-Pentyl-, Isopentyl-, Neopentyl-, n-Hexyl-, Isohexyl- oder auch n-Heptylreste sein.

Eine Cycloalkylgruppe ist in erster Linie ein monocyclischer Rest mit 3—10 Kohlenstoffatomen, vorzugsweise mit 5—7 Kohlenstoffatomen und ist z.B. eine Cyclopropyl-, Cyclobutyl-, vorzugsweise eine Cyclopentyl-, Cyclohexyl- oder auch Cycloheptylgruppe.

Die beiden Substituenten $R_1$ und $R_2$ zusammengenommen können einen gegebenenfalls durch Niederalkyl oder gegebenenfalls substituiertes Phenyl substituierten bivalenten aliphatischen Kohlenwasserstoffrest mit 4—7 Kohlenstoffatomen in der Kette bedeuten. Die Gruppe $—NR_1R_2$ ist beispielsweise Niederalkylenamino, in der die Niederalkylenkette beispielsweise durch ein Heteroatom, wie z.B. Sauerstoff, Schwefel oder gegebenenfalls durch Niederalkyl, gegebenenfalls substituiertes Phenyl, Benzyl, Phenyläthyl oder Alkoxycarbonyl, wie z.B. Methoxy- oder Aethoxycarbonyl substituierten Stickstoff unterbrochen sein kann, und stellt z.B. Niederalkylenamino, z.B. Pyrrolidino, 2,5-Dimethylpyrrolidino, Piperidino, 2-Methyl-, 4-Methyl- oder 4-Phenylpiperidino, Hexahydroazepino oder Octahydroazocino, Oxaniederalkylenamino, z.B. Morpholino, 2,6-Dimethylmorpholino, Thianiederalkylenamino, z.B. Thiomorpholino oder 2,6-Dimethylthiomorpholino und Azaniederalkylenamino, z.B. Piperazino, N-Methyl-, N-Phenyl-, N-Benzyl-, N-Methoxycarbonyl oder N-Aethoxycarbonylpiperazin dar.

Ph oder ein vorstehend genannter gegebenenfalls substituierter Phenylrest kann durch einen, zwei oder mehrere gleiche oder verschiedene Substituenten substituiert sein.

Solche Substituenten sind wie unter Formel I für Ph genannt, z.B. Niederalkyl, Hydroxy oder Mercapto, Niederalkoxy, Niederalkenyloxy oder Niederalkylendioxy, ferner Niederalkylthio oder Halogen, Trifluormethyl, Nitro, Amino, Niederalkylamino oder Di-niederalkylamino, Sulfamyl, Niederalkylsulfamyl oder Diniederalkylsulfamyl, Carboxy, Niederalkoxycarbonyl.

Die als Substituenten in Frage kommenden Niederalkylreste werden vorstehend für $R_1$, $R_2$ und $R_3$ definiert.

Niederalkoxy ist z.B. Methoxy, Aethoxy, n-Propoxy, Isopropoxy, n-Butoxy oder n-Pentyloxy, und Niederalkenyloxy z.B. Vinyloxy oder Allyloxy.

Halogenatome sind in erster Linie Fluor-, Chlor- oder Bromatome, können aber auch Jodatome sein.

Niederalkylthio ist insbesondere Methylthio, ferner auch Aethylthio, Isopropylthio, n-Propylthio oder auch gerades oder verzweigtes Butylthio.

Niederalkylamino oder Diniederalkylamino ist z.B. Methylamino, Dimethylamino, Aethylamino, Diäthylamino, n-Propylamino, Di-n-propylamino, Isopropylamino, Di-isopropylamino oder n-Butylamino oder Di-n-butylamino.

Niederalkyl- oder Diniederalkylsulfamyl ist z.B. Methylsulfamyl, Dimethylsulfamyl, Aethylsulfamyl, Diäthylsulfamyl, n-Propylsulfamyl, Di-n-propylsulfamyl, Isopropylsulfamyl, Diisopropylsulfamyl, n-Butylsulfamyl oder Di-n-butylsulfamyl.

Die neuen Verbindungen gemäss vorliegender Erfindung können aufgrund der vorliegenden Tautomerie, im Falle der Bedeutung für $R_3$ = Wasserstoff, tautomer vorliegen. Die Tautomeren können durch die Formeln

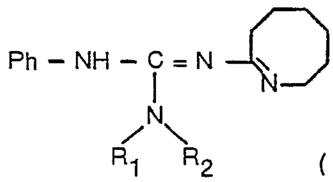
(Ia)

oder

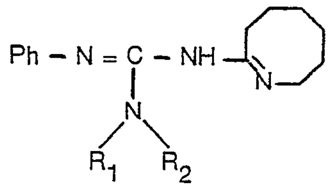
(Ib) .

wiedergegeben werden.

Die neuen Verbindungen der allgemeinen Formel I und ihre Additionssalze mit anorganischen oder organischen Säuren besitzen wertvolle pharmakologische Eigenschaften, insbesondere hypoglykämische Wirksamkeit, wie sich an Stoffwechsel-normalen Ratten nach oraler Verabreichung von Dosen ab 10 mg/kg sowie auch an Ratten, die durch Injektion von Streptozotocin in eine Diabetes-ähnliche Stoffwechsellage versetzt wurden [vgl. A. Junod et al., Proc. Soc. Exp. Biol. Med. *126*, 201—205 (1967)], nachweisen lässt. Die Senkung des Blutzuckerspiegels ist nicht von einer Hyperlactatämie begleitet. Analoge Wirkungen können auch am Meerschweinchen, Hamster und Rhesusaffen nachgewiesen werden. Die pharmakologischen Befunde charakterisieren die neuen Verbindungen der allgemeinen Formel I und ihre pharmazeutisch annehmbaren Säureadditionssalze als Antidiabetica, die zur oralen Behandlung von Hyperglykämie bei Säugetieren, insbesondere von Diabetes mellitus, verwendet werden können.

Die Erfindung betrifft in erster Linie Verbindungen der Formel I, in denen Ph einen gegebenenfalls substituierten Phenylrest, $R_1$ und $R_2$ unabhängig voneinander einen Niederalkylrest, beispielsweise Methyl- oder Aethyl- oder Cycloalkyl-, wie z.B. Cyclopentyl-, Cyclohexyl- oder Cycloheptyl-, oder beide zusammengenommen einen gegebenenfalls durch Niederalkyl oder Phenyl substituierten bivalenten Kohlenwasserstoffrest aliphatischen Charakters mit 4—7 Kohlenstoffatomen in der Kette, in welchem die Kohlenstoffatome der Kette durch ein Heteroatom unterbrochen sein können, $R_3$ Wasserstoff oder Niederalkyl bedeuten, ihre tautomeren Verbindungen und Salze.

Insbesondere betrifft die Erfindung diejenigen Verbindungen der Formel I, in denen Ph ein gegebenenfalls durch Niederalkyl, Niederalkoxy, Halogen oder Trifluormethyl substituiertes Phenyl, $R_1$ und $R_2$ beide zusammengenommen eine gegebenenfalls durch Niederalkyl oder Phenyl substituierte Niederalkylenkette, in welcher die Kohlenstoffatome der Kette durch ein Heteroatom, wie z.B. Sauerstoff, Schwefel oder gegebenenfalls durch Niederalkyl, Phenyl, Benzyl, Phenyläthyl oder auch Alkoxycarbonyl substituierten Stickstoff unterbrochen sein können, und $R_3$ Wasserstoff oder Niederalkyl bedeuten, ihre tautomeren Verbindungen und Salze.

Von besonderem Interesse sind Verbindungen der Formel I, in denen Ph ein gegebenenfalls durch Niederalkyl, Niederalkoxy, Halogen oder Trifluormethyl substituiertes Phenyl bedeutet, die Gruppe $—NR_1R_2$ beispielsweise ein durch Niederalkyl oder Phenyl substituiertes Niederalkylenamino bedeutet, in der die Niederalkylenkette gegebenenfalls durch Sauerstoff, Schwefel oder gegebenenfalls durch Niederalkyl, Phenyl oder Alkoxycarbonyl substituierten Stickstoff unterbrochen sein kann, und beispielsweise Pyrrolidino, 2,5-Dimethylpyrrolidino, Piperidino, 2-Methyl-, 4-Methyl- oder 4-Phenylpiperidino, Hexahydroazepino, Morpholino, 2,6-Dimethylmorpholino, Thiomorpholino, 2,6-Dimethylthiomorpholino, Piperazino, N-Methyl-, N-Phenyl-, N-Benzyl-, N-Methoxy-, N-Aethoxycarbonylpiperazin sein kann, und $R_3$ Wasserstoff oder auch Niederalkyl bedeutet, ihre tautomeren Verbindungen und Salze.

Von ganz besonderem Interesse sind Verbindungen der Formel I, in denen Ph ein gegebenenfalls durch Niederalkyl, wie z.B. Methyl oder Aethyl, Niederalkoxy, wie z.B. Methoxy oder Aethoxy, Halogen, wie z.B. Chlor oder Brom oder Trifluormethyl substituiertes Phenyl bedeutet, die Gruppe $—NR_1R_2$ beispielsweise ein durch Niederalkyl, wie z.B. Methyl oder Aethyl, oder Phenyl substituiertes Niederalkylenamino bedeutet, in der die Niederalkylenkette gegebenenfalls durch Sauerstoff oder gegebenenfalls durch Niederalkyl, wie z.B. Methyl oder Aethyl, Alkoxycarbonyl, wie z.B. Methoxy- oder Aethoxycarbonyl substituierten Stickstoff unterbrochen sein kann, und beispielsweise Pyrrolidino, Piperidino, 4-Methyl- oder 4-Phenylpiperidino, Morpholino, 2,6-Dimethylmorpholino, Piperazino, N-Methyl, N-Methoxycarbonylpiperazin sein kann, und $R_3$ Wasserstoff oder auch Niederalkyl, wie z.B. Methyl oder Aethyl bedeutet, ihre tautomeren Verbindungen und Salze.

Die neuen Guanidine der Formel I werden nach an sich bekannten Methoden erhalten.

So kann man z.B. die neuen Verbindungen der Formel I erhalten, indem man eine Verbindung der Formel II

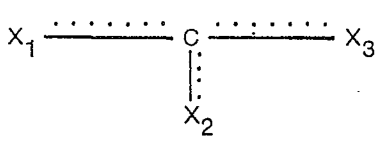
(II)

worin $X_1$ die Gruppe Ph—N=, in der Ph einen gegebenenfalls substituierten Phenylrest bedeutet, oder eine abspaltbare Gruppe, $X_2$ die Gruppe —$NR_1R_2$, worin $R_1$ und $R_2$ die unter der Formel I angegebene Bedeutung haben, oder eine abspaltbare Gruppe und $X_3$ die Gruppe

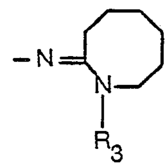

,

worin $R_3$ die unter der Formel I angegebene Bedeutung hat, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ oder $X_3$ mit dem Kohlenstoffatom durch eine Doppelbindung gebunden ist, mit einem Amin oder Imin umsetzt, welches der fehlenden Amino- oder Iminogruppe entspricht, die unter $X_1$, $X_2$ oder $X_3$ definiert werden, um die abspaltbare Gruppe zu ersetzen, und, wenn erwünscht, zusätzlichen Verfahrensschritte durchführt, und/oder, wenn erwünscht, erhaltene Verbindungen in ein Salz überführt, und/oder, wenn erwünscht, erhaltene Salze der Verbindungen der Formel I in die freien Basen umwandelt.

Als abspaltbare Gruppen $X_1$, $X_2$ oder $X_3$ werden, wie oben bereits angegeben, die durch eine Amino- oder Iminogruppe ersetzbare Gruppen bezeichnet, und bedeuten vorzugsweise Niederalkylthiogruppen, wie z.B. Methylthio oder Aethylthio, Niederalkoxy, wie z.B. Methoxy oder Aethoxy, oder auch Halogen, wie z.B. Chlor oder Brom.

Verbindungen der allgemeinen Formel II sind, je nachdem die abspaltbare Gruppe $X_1$, $X_2$ oder $X_3$ bedeutet, entweder Verbindungen der Formel IIa

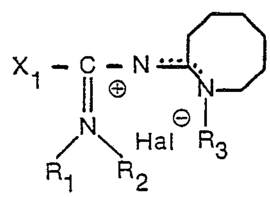

(IIa) ,

worin $X_1$ eine abspaltbare Gruppe, $R_1$, $R_2$ und $R_3$ die oben angegebene Bedeutungen haben, oder Verbindungen der Formel IIb

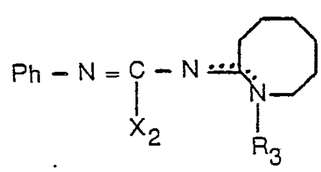

(IIb) ,

worin $X_2$ eine abspaltbare Gruppe, Ph, $R_3$ die oben angegebenen Bedeutungen haben, oder Verbindungen der Formel IIc,

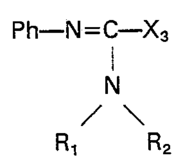

(IIc)

worin $X_3$ eine abspaltbare Gruppe, Ph, $R_1$ und $R_2$ die oben angegebenen Bedeutungen haben, ihre tautomeren Formen, oder ihre Säureadditionssalze.

Je nachdem als abspaltbare Gruppe $X_1$, $X_2$ oder $X_3$ in einer Verbindung der Formel II vorhanden ist, setzt man eine Verbindung der Formel IIb mit einem Amin der Formel $HNR_1R_2$, eine Verbindung der Formel IIc mit einer Iminoverbindung der Formel III

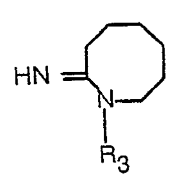

(III)

oder eine Verbindung der Formel IIa mit einem gegebenenfalls substituierten Anilin der Formel Ph—$NH_2$ um. Verbindungen der Formel IIa, IIb und IIc können auch als Säureadditionssalze, vorzugsweise als Hydrohalogenide verwendet werden. In analoger Weise können auch die verwendeten Amine, Iminoverbindungen oder auch Aniline als Säureadditionssalze, vorzugsweise als Hydrohalogenide umgesetzt werden.

Die Umsetzung einer Verbindung der Formel II, d.h. insbesondere einer Verbindung der Formel IIa, IIb oder IIc beispielweise mit einem vorher genannten Amin oder Imin als freie Base erfolgt unter Verwendung eines stöchiometrischen Ueberschusses des Amins oder Imins, beispielsweise in einem Molverhältnis 1:1,05 bis 1:2,0. Bei Verwendung eines nur geringen Ueberschusses des Amins oder Imins als freie Base oder bei Verwendung des Amins oder Imins als Säureadditionssalze ist es zweckmässig, eine stöchiometrisch äquivalente Menge eines teriären Alkylamins, wie z.B. Triäthylamin oder N-Aethyldiisopropylamin zusätzlich hinzuzusetzen.

Setzt man beispielsweise eine Iminoverbindung der Formel III als freie Base

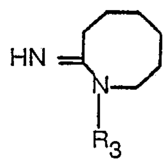

(III)

mit einer Verbindung der Formel IIc, in der $X_3$ ein Halogen bedeutet, um, so verwendet man vorzugsweise 2 Moläquivalente oder mehr der freien Base der oben erwähnten Iminoverbindung. Gemäss folgendem Reaktionsschema

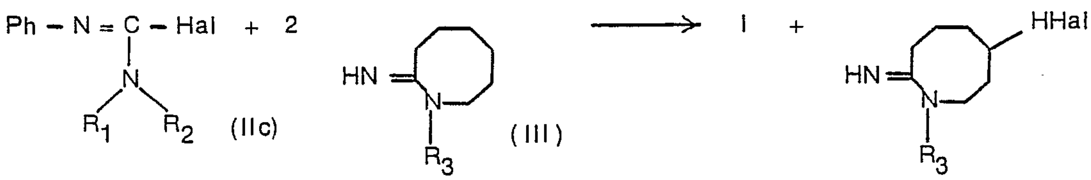

entsteht ein Aequivalent der Iminoverbindung als Säureadditionssalz. Aus diesem Grunde wird die Umsetzung vorzugsweise in einem aprotischen Lösungsmittel durchgeführt, in dem die erhaltene Verbindung der Formel I löslich, wogegen das Additionssalz der Halogenwasserstoffsäure gemäss obiger Formel als unlösliche Verbindung ausfällt. Auf diese Weise lassen sich die beiden erhaltenen Reaktionsprodukte leicht voneinander trennen.

Das erhaltene Säureadditionssalz der Iminoverbindung wird durch basische Hydrolyse, beispielsweise durch Zusatz von Alkali- oder Erdalkalihydroxyd oder -carbonat in die freie Base übergeführt und kann hiermit als Ausgangsprodukt zur Wiederverwendung zurückgewonnen werden. Vorzugsweise setzt man jedoch Verbindungen der Formel IIc und III als Säureadditionssalze, beispielsweise als Halogenide, wie oben angegeben in Gegenwart eines zusätzlichen tertiären Alkylamins, wie z.B. Triäthylamin oder N-Aethyldiisopropylamin um.

Die beschriebenen Umsetzungen von Verbindungen der Formel IIc mit einer Iminoverbindung der Formel III erfolgen, wie bereits erwähnt, vorzugsweise in aprotischen Lösungsmitteln. Beispiele für bevorzugt verwendbare Lösungsmittel sind Aether, wie z.B. Diäthyläther und Tetrahydrofuran, niedere aliphatische Ketone und Ester, wie z.B. Aceton, Methyläthylketon und Aethylacetat, aromatische Kohlenwasserstoffe, wie z.B. Benzol, Toluol oder Xylol, sowie Acetonitril. Besonders bevorzugt jedoch wird die Umsetzung in Diäthyläther oder Acetonitril ausgeführt. Die Umsetzungen können bei einer Temperatur zwischen 0—150°C, vorzugsweise jedoch zwischen Raumtemperatur und Rückflusstemperatur des Reaktionsgemisches ausgeführt werden.

Verwendet man als Ausgangsverbindung der Formel II jedoch zum Beispiel eine Verbindung der Formel IIb

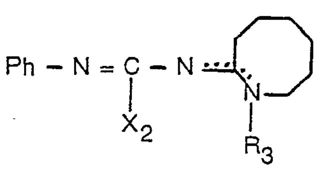

(IIb) ,

so hat $X_2$ als abspaltbare Gruppe vorzugsweise die Bedeutung einer Niederalkoxy- oder Niederalkylthiogruppe. Ausgangsverbindungen der Formel IIb werden in Form ihrer Salze, beispielsweise in Form ihrer Säureadditionssalze mit einer Halogenwasserstoffsäure mit einem Amin der Formel $HNR_1R_2$ als freie Base oder als Säureadditionssalz umgesetzt, in der $R_1$ und $R_2$ die vorstehend angegebene Bedeutung haben.

Die Umsetzungen werden z.B. in einem Alkohol als Lösungsmittel, vorzugsweise in einem niederen Alkanol, wie z.B. Aethanol, Isopropanol oder tert.-Butanol, besonders bevorzugt jedoch in einem Aether, wie z.B. Diäthyläther oder Tetrahydrofuran, oder in Acetonitril, bei Temperaturen von Raumtemperatur bis

vorzugsweise zur Rückflusstemperatur des Reaktionsgemisches durchgeführt. Die Reaktionen können jedoch in einem geschlossenen Reaktionsgefäss unter Druck, wie z.B. in einem Bombenrohr oder in einem Autoklaven, bei höheren Temperaturen ausgeführt werden. Die Guanidinderivate der allgemeinen Formel I werden in Form ihrer Salze erhalten, die beispielsweise durch alkalische Hydrolyse in die entsprechenden freien Basen überführt werden können. Bei der Umsetzung der Verbindungen der allgemeinen Formel IIb mit dem Amin der allgemeinen Formel $HNR_1R_2$ wird das Amin vorzugsweise in stöchiometrischem Ueberschuss verwendet, beispielsweise in einem Molverhältnis 1:1,05 bis 1:2,0 und mehr. Bei Verwendung nur eines geringen Ueberschusses des Amins oder eines Säureadditionssalzes kann es zweckmässig sein, eine zusätzliche stöchiometrische äquivalente Menge eines tertiären Alkylamins, wie z.B. Triäthylamin oder N-Aethyldiisopropylamin, zuzusetzen, um die Reaktionsgeschwindigkeit zu erhöhen.

Die Umsetzungen von Verbindungen der Formel IIa mit einer abspaltbaren Gruppe $X_1$, die neben der Bedeutung eines Halogenatoms, vorzugsweise Niederalkoxy oder Niederalkylthio bedeutet, oder ihrer tautomeren Form mit einem gegebenenfalls substituierten Anilin als freie Base erfolgen in gleicher Weise wie bei der Umsetzung einer Verbindung der Formel IIb mit einem Amin der Formel $HNR_1R_2$ beschrieben. Die Umsetzungen werden vorteilhafterweise auch in einem stöchiometrischen Ueberschuss der gegebenenfalls substituierten Aniline ausgeführt. Bei Verwendung nur eines geringen Ueberschusses des Anilins oder eines Säureadditionssalzes davon kann es zweckmässig sein, eine stöchiometrisch äquivalente Menge eines bereits oben definierten tertiären Trialkylamins zuzusetzen. Die Umsetzungen werden in analogen Lösungsmitteln, wie vorher bei der Umsetzung der Verbindungen IIc mit Verbindungen der Formel III beschrieben, ausgeführt.

Verbindungen der allgemeinen Formel I können auch hergestellt werden, indem man eine Guanidinverbindung der allgemeinen Formel IV

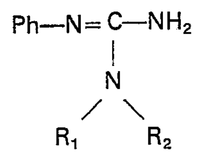

(IV),

worin Ph, $R_1$ und $R_2$ die unter der Formel I angegebenen Bedeutungen haben, mit einer Verbindung der allgemeinen Formel V

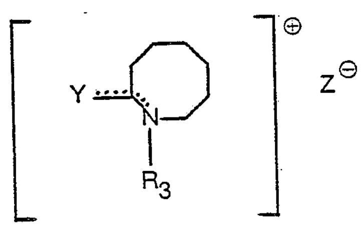

(V) ,

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, wie z.B. Methylsulfat- oder Niederalkansulfonat-, wie z.B. Methansulfonatanion oder ein Halogenid, wie z.B. Chlorid oder Bromid, bedeutet, wobei, wenn Y die Bedeutung von zwei Niederalkoxygruppen am gleichen C-Atom hat, Z als Anion entfällt, oder, wenn $R_3$ Wasserstoff bedeutet, die tautomere Form als freie Base vorliegt, umsetzt, und, wenn erwünscht, erhaltene Verbindungen der Formel I in ein Salz überführt, und/oder, wenn erwünscht, erhaltene Salze der Verbindungen der Formel I in die freien Verbindungen überführt.

Die Verbindungen der allgemeinen Formel I werden zweckmässig in der Weise hergestellt, dass man ein Lactamsalz der vorstehend angegebenen Formel V mit einem Guanidinderivat der oben definierten Formel IV in stöchiometrischen Mengen zur Umsetzung bringt. Die Umsetzungen werden vorzugsweise in einem wasserfreien organischen Lösungsmittel durchgeführt. Organische Lösungsmittel sind z.B. niedere Alkanole, wie z.B. Methanol, Aethanol, Isopropanol, tert.-Butanol, Aether, wie z.B. Diäthyläther, Tetrahydrofuran oder Dioxan, niedere halogenierte Kohlenwasserstoffe, wie z.B. Chloroform, Methylenchlorid oder 1,2-Dichloräthan, und aromatische Kohlenwasserstoffe, wie z.B. Benzol, Toluol oder Xylol. Im allgemeinen wird die Reaktion bei Temperaturen, die zwischen $-20°C$ und $+50°C$ liegen, vorzugsweise jedoch zwischen 0°C und Raumtemperatur durchgeführt.

Das in Salzform erhaltene Reaktionsprodukt der allgemeinen Formel I wird durch basische Hydrolyse, beispielsweise durch Zusatz von einem Alkali- oder Erdalkalihydroxyd oder -carbonat in die freie Base übergeführt.

Die verfahrensgemäss eingesetzten Lactamfluoroborate oder -fluorsulfonate der allgemeinen Formel V, in der $Z^{\ominus}$ beispielsweise die Tetrafluorborat-Gruppe der Formel $BF_4^{\ominus}$ oder Fluorsulfonatgruppe der Formel $OSO_2F^{\ominus}$ darstellt, können nach üblichen Verfahren hergestellt werden, indem man ein Lactam der Formel Va

mit einem entsprechenden Trialkyloxoniumfluoroborat oder einem Fluorsulfonsäureniederalkylester zu dem entsprechenden Lactamsalz der allgemeinen Formel V umsetzt.

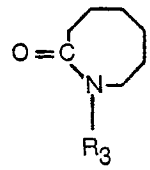

(Va)

Die Umsetzung wird beispielsweise bei Temperaturen zwischen −20°C und +50°C, vorzugsweise bei Temperaturen zwischen 0°C und +25°C in einem inerten Gas, wie z.B. Stickstoff oder Argon, und in Gegenwart eines inerten, wasserfreien organischen Lösungmittels, beispielsweise in einem niederen Halogenkohlenwasserstoff, wie z.B. Chloroform, 1,2-Dichloräthan oder vorzugsweise Methylenchlorid durchgeführt. Beispiele für andere verwendbare organische Lösungsmittel sind Aether, wie z.B. Diäthyläther, Dioxan, Tetrahydrofuran oder 1,2-Dimethoxyäthan, sowie aromatische Kohlenwasserstoffe, wie z.B. Benzol, Toluol oder Xylol.

Die unter die allgemeine Formel V fallenden 2-Niederalkylthiolactimäther können durch Umsetzung des Lactams der allgemeinen Formel Va mit Phosphorpentasulfid in analoger Weise nach dem von R. Gomper et al., Org. Syn. Coll., Vol. V, Seiten 780—785 beschriebenen Verfahren hergestellt werden. Bei Ausführung dieser Umsetzung erhält man zunächst ein Thiolactam, welches durch Umsetzung mit einem Alkylierungsmittel den 2-Alkylthiolactimäther in Form der entsprechenden Salze liefert. Als Alkylierungsmittel kann ein Alkylhalogenid, beispielsweise Methyljodid, ein Fluorsulfonsäurealkylester, wie z.B. Fluorsulfonsäuremethylester, ein Methansulfonsäurealkylester, wie z.B. Methansulfonsäuremethylester, ein Toluolsulfonsäurealkylester, wie z.B. Toluolsulfonsäuremethylester, oder Dimethylsulfat verwendet werden. Die Umsetzung der Lactimäthersalze mit dem Guanidinderivat der allgemeinen Formel IV liefert die entsprechenden Salze der allgemeinen Formel I.

Bei der Umsetzung der vorher beschriebenen Lactamfluorsulfonate der allgemeinen Formel V mit dem Guanidinen der allgemeinen Formel IV können als Nebenreaktion auch quaternäre Ammoniumsalze der Verbindungen der allgemeinen Formel I entstehen.

Die auch unter die allgemeine Formel V fallenden Methylsulfatsalze werden in analoger Weise wie von H. Bredereck et al., Chem. Ber. Bd. 96 (1963), S. 1350 für Pyrrolidone beschrieben, aus Lactamen der allgemeinen Formel Va durch Umsetzung mit Dimethylsulfat erhalten. Die Umsetzung wird vorzugsweise in einem wasserfreien, inerten organischen Lösungsmittel, beispielsweise einem aromatischen Kohlenwasserstoff, wie z.B. Benzol, Toluol oder Xylol, einem Aether, wie z.B. Diäthyläther, Dioxan oder Tetrahydrofuran, oder einem halogenierten aliphatischen Kohlenwasserstoff, wie z.B. 1,2-Dichloräthan oder Chloroform durchgeführt. Das erhaltene Methosulfat der allgemeinen Formel V wird dann mit dem entsprechenden Guanidinderivat der allgemeinen Formel IV auf vorstehend beschriebene Weise zum entsprechenden Niederalkylsulfatsalz, wie z.B. Methylsulfatsalz der Verbindung der allgemeinen Formel I überführt. Die erhaltenen Salze lassen sich durch Behandlung mit einem Alkali- oder Erdalkalihydroxyd oder -carbonat in entsprechende freie Basen der allgemeinen Formel I umwandeln.

Aus dem Niederalkylsulfat-, wie z.B. Methylsulfatsalz der allgemeinen Formel V lässt sich beispielsweise durch Umsetzung mit einem Metallalkoxid, vorzugsweise einem Alkalimetallalkoxid, wie z.B. Natriummethoxid oder -äthoxid, im entsprechenden wasserfreien niederen Alkanol das entsprechende Lactamacetal der Formel Vb

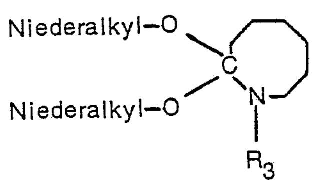

(Vb)

herstellen.

Aus den Lactamacetalen lassen sich, wie vorstehend beschrieben, mit den Guanidinderivaten der allgemeinen Formel IV die freien Basen der allgemeinen Formel I herstellen.

Die verfahrensgemäss verwendeten Halogenid-, insbesondere Chloridsalze der Lactame der allgemeinen Formel V lassen sich in analoger Weise wie von W. Jentsch und M. Seefelder, Chem. Ber., Bd. 98 (1965), S. 274 für Pyrrolidone beschrieben, durch Umsetzung eines Lactams der allgemeinen Formel Va mit Phosgen oder Thionylchlorid herstellen.

Wie bereits vorstehend erwähnt, können für die Herstellung der Verbindungen der allgemeinen Formel I, in der $R_3$ ein Wasserstoffatom bedeutet, auch die freien Basen der allgemeinen Formel V verwendet werden. Die Umsetzung der Salze der allgemeinen Formel V mit einer Base, wie z.B. einem Alkali- oder Erdalkalihydroxyd- oder -carbonat, vorzugsweise in einem halogenierten aliphatischen Kohlenwasserstoff als Lösungsmittel, wie z.B. Methylenchlorid oder Chloroform, liefert die freien Basen der allgemeinen Formel Vc

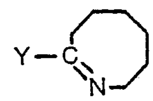

(Vc)

Die erfindungsgemässen Verbindungen der Formel I, in der $R_1$ die oben definierte Bedeutung hat und $R_2$ und/oder $R_3$ Wasserstoff bedeuten, können nach einem dritten Verfahren durch Umsetzung mit einem reaktionsfähigen Ester eines Niederalkanols oder gegebenenfalls Cycloalkanols in Verbindungen der Formel I umgewandelt werden, in denen $R_2$ und/oder $R_3$ im Rahmen der vorstehenden Definition für $R_2$ und $R_3$ verschieden von Wasserstoff sind, und, wenn erwünscht, können zusätzliche Verfahrensschritte durchgeführt, und/oder, wenn erwünscht, erhaltene Verbindungen der Formel I in ein Salz übergeführt, und/oder, wenn erwünscht, erhaltene Salze der Verbindungen der Formel I in die freien Basen umgewandelt werden.

Als reaktionsfähigen Ester, als sogenanntes Alkylierungsmittel kann man z.B. ein Niederalkyl- oder Cycloalkylhalogenid wie z.B. Methyl- oder Cyclohexyliodid, einen entsprechenden Fluorsulfonsäurealkylester, wie z.B. Fluorsulfonsäuremethylester, einen entsprechenden Niederalkansulfonsäureester, wie z.B. Methansulfonsäuremethylester, einen entsprechenden Toluolsulfonsäurealkylester, wie z.B. Toluolsulfonsäuremethylester, oder ein Dialkylsulfat, wie z.B. Dimethyl- oder Diäthylsulfat, verwenden.

Die erfindungsgemässen Verbindungen der Formel I, in denen $R_2$ und/oder $R_3$ Wasserstoff bedeuten, können nach einem vierten Verfahren hergestellt werden, indem man in Verbindungen der allgemeinen Formel VI

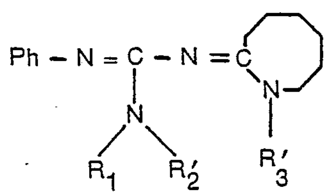

(VI),

worin $R_1$ und Ph die unter der Formel I angegebene Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

Eine Aminoschutzgruppe $R_2'$ bzw. $R_3'$ ist in erster Linie eine Acylgruppe, wie Acyl einer aliphatischen, aromatischen oder araliphatischen Carbonsäure, insbesondere Niederalkanoyl, z.B. Acetyl oder Propionyl, oder Aroyl, z.B. Benzoyl oder Acyl der Ameisensäure oder eines Kohlensäurehalbderivates, z.B. -esters, wie Formyl, Niederalkoxycarbonyl, z.B. Aethoxycarbonyl oder tert.-Butyloxycarbonyl, oder Arylniederalkoxycarbonyl, z.B. Benzyloxycarbonyl.

Die Abspaltung eines als Aminoschutzgruppe $R_2'$ und/oder $R_3'$ verwendeten Acylrestes erfolgt in an sich bekannter Weise, z.B. durch Solvolyse, in erster Linie mittels Alkoholyse, ferner mittels Hydrolyse. Die alkoholytische Abspaltung eines Acylrestes $R_2'$ und/oder $R_3'$ kann z.B. in Gegenwart eines stark basischen Mittels, bei erhöhter Temperatur, z.B. bei etwa 50°C bis etwa 120°C, erfolgen. Dabei verwendet man insbesondere einen Niederalkanol, z.B. n-Butanol oder Aethanol, und als starke Base ein Alkalimetall-, z.B. Natrium- oder Kalium-niederalkanolat, z.B. -n-butylat oder -äthylat, oder ein Alkalimetallhydroxid, z.B. Natrium- oder Kaliumhydroxid.

Aminoschutzgruppen $R_2'$ und $R_3'$, beispielsweise Niederalkoxycarbonylgruppen, wie tert.-Butyloxycarbonyl, lassen sich besonders schonend acidolytisch, z.B. durch Behandeln mit Trifluoressigsäure, abspalten.

Eine weitere, besonders schonend abspaltbare Aminoschutzgruppe ist eine Aethoxycarbonylgruppe, die in β-Stellung eine mit drei Kohlenwasserstoffresten substituierte Silylgruppe, wie Triphenylsilyl-, Dimethyl-butyl-silyl- oder vor allem Trimethylsilylgruppe, trägt. Eine solche β-(Trimethylsilyl)-äthoxycarbonylgruppe bildet mit der zu schützenden Aminogruppe eine entsprechende β-Trimethylsilyl-äthoxycarbonylaminogruppe, welche sich unter milden Bedingungen durch Einwirkung von Fluoridionen abspalten lässt. Als Fluoridionen-abgebende Reagentien kommen beispielsweise Fluoride quaternärer organischer Basen, wie Tetraäthylammoniumfluorid in Frage.

Es ist dabei zu beachten, dass als Aminoschutzgruppe $R_2'$ und/oder $R_3'$ nur solche in Frage kommen, die selektiv unter Erhaltung der Struktur der Verbindungen der allgemeinen Formel I abspaltbar sind.

Die Ausgangsstoffe sind bekannt, oder falls sie neu sind, lassen sie sich nach an sich bekannten Methoden herstellen. Wo es sich als zweckdienlich erweist, sind die verwendeten Ausgangsprodukte im Anschluss an das beschriebene Verfahren bereits beschrieben worden.

Verbindungen der allgemeinen Formel IIb, in der $X_2$ eine Niederalkylthiogruppe bedeutet, lassen sich beispielsweise aus den entsprechenden Thioharnstoffen der allgemeinen Formel VII

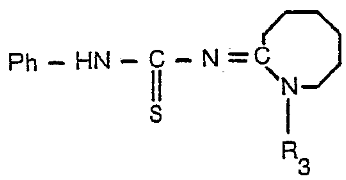

(VII)

herstellen, indem man diese mit einem vorstehend aufgezählten reaktionsfähigen Ester eines Niederalkanols umsetzt.

Die Umsetzung wird in einem bereits vorstehend definierten organischen Lösungsmittel ausgeführt. Vorzugsweise verwendet man als Lösungsmittel einen Aether, wie z.B. Diäthyläther, Tetrahydrofuran oder Dioxan, ein Keton, wie z.B. Aceton oder 2-Butanon, einen halogenierten aliphatischen Kohlenwasserstoff, wie z.B. Chloroform oder Methylenchlorid oder einen niederen Alkanol, wie z.B. Methanol oder Aethanol. Besonders geeignet ist ein Alkylhalogenid in Methanol bzw. Aethanol. Im allgemeinen wird das Alkylierungsmittel in mindestens äquimolarer Menge verwendet. Die Alkylierung kann gegebenenfalls bei Raumtemperatur oder bei höheren Temperaturen und nötigenfalls in einem geschlossenen Reaktionsgefäss durchgeführt werden.

Verbindungen der allgemeinen Formel VII wiederum lassen sich aus den bereits erwähnten und bekannten Iminoverbindungen der Formel III

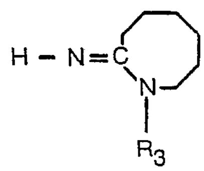

(III)

durch Umsetzung mit einem gegebenenfalls substituierten Phenylisothiocyanat der Formel Ph—NCS in einem bereits vorstehend definierten inerten organischen Lösungsmittel, vorzugsweise in Benzol, Methylenchlorid oder Chloroform, bei Temperaturen von 0°C bis Raumtemperatur während 2—24 Stunden in etwa äquimolaren Mengen herstellen.

Verbindungen der Formel IIc, in der $X_3$ als abspaltbare Gruppe Halogen, vorzugsweise Chlor bedeutet, werden nach der von E. Kühle, Angew. Chem., Intern. Ed., Bd. 8 (1969), S. 24—26 beschriebenen Methode erhalten, indem man ein Isocyaniddihalogenid der Formel VIII

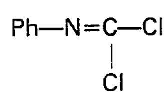

(VIII)

mit einem Amin der Formel $HNR_1R_2$, in Gegenwart eines Trialkylamins, wie z.B. Triäthylamin, in einem inerten aprotischen, wasserfreien Lösungsmittel umsetzt. Verbindungen der Formel VIII können auch als Immoniumchloride vorliegen. Als Lösungsmittel verwendet man z.B. einen Aether, beispielsweise Diäthyläther, Dioxan oder Tetrahydrofuran, einen halogenierten aliphatischen Kohlenwasserstoff, wie z.B. Chloroform oder Methylenchlorid, oder einen aromatischen Kohlenwasserstoff, wie z.B. Benzol, Toluol oder Xylol. Verbindungen der allgemeinen Formel VIII sind bekannt und lassen sich in analoger Weise, wie in Angew. Chem., Intern. Ed. Bd. 6 (1967), S. 649 beschrieben, herstellen.

Verbindungen der allgemeinen Formel IIc, in der die abspaltbare Gruppe $X_3$ Halogen bedeutet, lassen sich leicht auf bekannte Weise in Verbindungen der Formel IIc umwandeln, in der $X_3$ eine Niederalkoxygruppe bedeutet.

Ausgangsverbindungen der allgemeinen Formel IIa, in der als abspaltbare Gruppe $X_1$ ein Halogen, vorzugsweise Chlor, bedeutet, lassen sich durch Umsetzung eines Immoniumchlorids der Formel

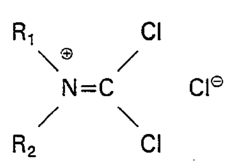

mit einer Iminoverbindung der Formel III

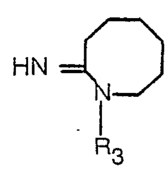

(III)

nach der von R. G. Glushkow, Khim.-Farmasevt. Zh. 12, No. 6, 59—64/1978 beschriebenen Methode herstellen.

Die Umsetzung erfolgt in analoger Weise wie oben bei der Umsetzung einer Verbindung der Formel VIII beschrieben.

Verbindungen der allgemeinen Formel VI lassen sich nach einem der für die Herstellung von Verbindungen der allgemeinen Formel I vorstehend beschriebenen Verfahren herstellen, wobei jedoch in den verwendeten Ausgangsprodukten $R_2$ und/oder $R_3$ einen Acylrest bedeuten. Diese als Aminoschutzgruppen verwendeten Acylreste sind wie oben definiert.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der Atmosphäre eines inerten Gases, wie z.B. Stickstoff, erfolgen.

In erhaltenen Verbindungen kann man im Rahmen der Definition der Endprodukte Substituenten einführen, abwandeln oder abspalten.

Je nach den Verfahrensbedingungen und Ausgangsstoffen erhält man die Endstoffe in freier Form oder in der ebenfalls in der Erfindung inbegriffenen Form ihrer Salze, insbesondere Säureadditionssalze. Die Säureadditionssalze der neuen Verbindungen können in an sich bekannter Weise in die freie Verbindung übergeführt werden, z.B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Trypthophan, Lysin oder Arginin.

Diese oder andere Salze der neuen Verbindungen, wie z.B. die Pikrate, können auch zur Reinigung der erhaltenen freien Basen dienen, indem man die freien Basen in Salze überführt, diese abtrennt und aus den Salzen wiederum die Basen freimacht. Infolge der engen Beziehungen zwischen den neuen Verbindungen in freier Form und in Form ihrer Salze sind im Vorausgegangenen und nachfolgend unter den freien Verbindungen sinn- und zweckmässig, gegebenenfalls auch die entsprechenden Salze zu verstehen.

Die Erfindung betrifft auch diejenigen Ausführungsformen eines Verfahrens, bei denen man ein Verfahren auf irgendeiner Stufe abbricht oder bei denen man von einer auf irgendeiner Stufe als Zwischenprodukt erhältlichen Verbindung ausgeht und die fehlenden Schritte durchführt, oder einen Ausgangsstoff unter den Reaktionsbedingungen bildet oder gegebenenfalls in Form eines Salzes verwendet. Die Erfindung beinhaltet auch daraus resultierende neue Zwischenprodukte.

Von der Erfindung ebenfalls umfasst sind therapeutische Stoffzusammenstellungen, die aus einem hypoglykämisch wirksamen Anteil der Verbindungen der allgemeinen Formel I oder einem Säureadditionssalz und einem pharmakologisch annehmbaren festen Trägerstoff oder flüssigen Verdünnungsmittel bestehen.

Die erfindungsgemässen pharmazeutischen Präparate enthalten mindestens eine Verbindung der allgemeinen Formel I oder ein Salz davon als Wirkstoff zusammen mit einem üblichen pharmazeutischen Trägerstoff. Die Art der Trägerstoffe richtet sich weitgehend nach dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung von Hyperglykämie kommen insbesondere feste Doseneinheitsformen, wie Tabletten, Dragées und Kapseln, in Frage, die vorzugsweise zwischen 10 und 90% eines Wirkstoffes der allgemeinen Formel I oder eines Salzes enthalten, um die Verabreichung von täglichen Dosen zwischen 1,5 bis 100 mg/kg an Warmblütern zu ermöglichen. Zur Herstellung von Tabletten und Dragéekernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschliessend beispielsweise mit konzentrierten Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Weiche Gelatinekapseln und andere geschlossene Kapseln bestehen beispielsweise aus einem Gemisch von Gelatine und Glycerin und können z.B. Mischungen einer Verbindung der Formel I mit Polyäthylenglykol enthalten. Steckkapseln enthalten z.B. Granulate eines Wirkstoffes mit festen, pulverförmigen Trägerstoffen, wie z.B. Lactose, Saccharose, Sorbit, Mannit; Stärken, wie Kartoffelstärke, Maisstärke oder Amylopektin, Cellulosederivate sowie Magnesiumstearat oder Stearinsäure.

Als Doseneinheitsformen für die rektale Anwendung kommen z.B. Suppositorien in Betracht, welche

aus einer Kombination eines Wirkstoffes mit einer Suppositorien-Grundmasse auf der Basis von natürlichen oder synthetischen Triglyceriden (z.B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Ampullenlösungen zur parenteralen, insbesondere intramuskulären oder intravenösen Verabreichung enthalten eine Verbindung der Formel I oder ein Salz davon in einer Konzentration von vorzugsweise 0,5 bis 5% als wässrige, mit Hilfe von üblichen Lösungsvermittlern und/oder Emulgiermitteln, sowie gegebenenfalls von Stabilisierungsmitteln bereitete Dispersion, oder vorzugsweise eine wässrige Lösung eines pharmazeutisch annehmbaren, wasserlöslichen Säureadditionssalzes einer Verbindung der allgemeinen Formel I.

Für Flüssigkeiten zur oralen Einnahme, wie Sirups und Elixiere, wird die Konzentration des Wirkstoffes derart gewählt, dass eine Einzeldosis leicht abgemessen werden kann, z.B. als Inhalt eines Teelöffels oder eines Messlöffels von z.B. 5 ml, oder auch als Mehrfaches dieser Volumina.

Die nachfolgenden Beispiele a) bis e) sollen die Herstellung einiger typischer Applikationsformen erläutern, jedoch keineswegs die einzigen Ausführungsformen von solchen darstellen.

a) 250,0 g Wirkstoff werden mit 550,0 g Lactose und 292,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und presst die Mischung zu 10'000 Tabletten von je 125 mg Gewicht und 25 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10'000 Dragéekernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und enthalten je 10 mg Wirkstoff.

c) 25,0 g Wirkstoff und 1975 g fein geriebene Suppositoriengrundmasse (z.B. Kakaobutter) werden gründliche gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25% Wirkstoffgehalt löst man in 3 Litern dest. Wasser 1,5 Liter Glycerin, 42 g p-Hydroxybenzoesäuremethylester, 18 g p-Hydroxybenzoesäure-n-propylester und unter leichtem Erwärmen 25,0 g Wirkstoff, fügt 4 Liter 70%-ige Sorbitlösung, 1000 g krist. Saccharose, 350 g Glucose und einen Aromastoff, z.B. 250 g "Orange Peel Soluble Fluid" von Eli Lilly and Co., Indianapolis, oder 5 g natürliches Zitronenaroma und 5 g "Halb und Halb"-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5% Wirkstoffgehalt löst man 150,0 g Wirkstoff und 30 g Natriumcyclamat in einem Gemisch von 4 Liter Aethanol (96%) und 1 Liter Propylenglykol. Andererseits mischt man 3,5 Liter 70%-ige Sorbitlösung mit 1 Liter Wasser und fügt die Mischung zur obigen Wirkstofflösung. Hierauf wird ein Aromastoff, z.B. 5 g Hustenbonbon-Aroma oder 30 g Grapefruit-Essenz, beide von der Firma Haarmann und Reimer, Holzminden, Deutschland zugegeben, das Ganze gut gemischt, filtriert und mit dest. Wasser auf 10 Liter ergänzt.

Die nachfolgenden Beispiele erläutern die Herstellung der neuen Verbindungen der allgemeinen Formel I, sollen jedoch den Umfang der Erfindung in keiner Weise beschränken. Die Temperaturen sind in Celsiusgraden angegeben.

Beispiel 1

Eine Suspension von 8 g N-Phenyl-1-pyrrolidin-carboximid-amid-hydrojodid in 30 ml Acetonitril wird mit 4 g 3,4,5,6,7,8-Hexahydro-2-methoxyazocin versetzt. Das Gemisch wird 12 Stunden unter starkem Rühren auf dem Wasserbad erhitzt. Das Acetonitril wird unter vermindertem Druck abgedampft und der Rückstand aus einem Gemisch von Aceton-Essigsäureäthylester umkristallisiert. Man erhält das N-Hexahydro-2(1H)-azocinyliden-N'-phenyl-1-pyrrolidincarboximidamid-hydrojodid der Formel

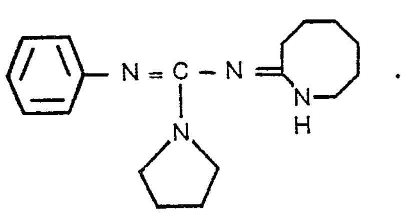 . HJ ,

welches bei 242° schmilzt.

Der Ausgangsstoff für die obige Synthese wird wie folgt hergestellt: Eine Suspension von 15 g N-Phenyl-S-methyl-isothio-harnstoff-hydrojodid in 50 ml Acetonitril wird mit 7 g Pyrrolidin versetzt. Das Gemisch wird unter Rühren 15 Stunden unter Rückfluss gekocht. Das Acetonitril wird unter vermindertem Druck abgedampft und der Rückstand aus einem Gemisch von Isopropanol-Essigsäureäthylester

umkristallisiert. Man erhält das N-Phenyl-1-pyrrolidincarboximidamid-hydrojodid, welches bei 165° schmilzt.

Beispiel 2

Eine Suspension von 6 g N-Phenyl-1-piperidincarboximidamid-hydrojodid in 20 ml Acetonitril wird mit 3 g 3,4,5,6,7,8-Hexahydro-2-methoxyazocin versetzt. Das Gemisch wird auf dem Wasserbad 12 Stunden unter Rückfluss gekocht und stark gerührt. Das Acetonitril wird unter vermindertem Druck abgedampft und der Rückstand aus einem Gemisch von Isopropanol-Essigsäureäthylester umkristallisiert. Man erhält das N-Hexahydro-2(1H)-azocinyliden-N'-phenyl-1-piperidincarboximidamid-hydrojodid, welches bei 205° schmilzt.

Der Ausgangsstoff wird wie folgt hergestellt: Eine Suspension von 15 g N-Phenyl-S-methyl-isothioharnstoff-hydrojodid in 50 ml Acetonitril wird mit 8 g Piperidin versetzt. Das Gemisch wird unter Rühren 15 Stunden unter Rückfluss gekocht. Das Acetonitril wird unter vermindertem Druck abgedampft und der Rückstand aus einem Gemisch von Isopropanol-Essigsäureäthylester umkristallisiert. Man erhält das N-Phenyl-1-piperidincarboximidamid-hydrojodid, welches bei 135° schmilzt.

Beispiel 3

Eien Suspension von 10 g N-Phenyl-4-morpholin-carboximidamid-hydrojodid in 15 ml Acetonitril wird mit 5,6 g 3,4,5,6,7,8-Hexahydro-2-methoxyazocin versetzt. Das Gemisch wird auf dem Wasserbad unter starkem Rühren 12 Stunden erhitzt. Das Acetonitril wird unter vermindertem Druck abgedampft und der Rückstand aus einem Gemisch von Aceton-Essigsäureäthylester umkristallisiert. Man erhält das N-Hexahydro-2(1H)-azocinyliden-N'-phenyl-4-morpholin-carboximidamid-hydrojodid, welches bei 260° schmilzt.

Die entsprechende Base und ihre Salze werden wie folgt hergestellt: Eine Suspension von 4 g N-Hexahydro-2(1H)-azocinyliden-N'-phenyl-4-morpholin-carboximidamid-hydrojodid in 50 ml Methylenchlorid wird unter Rühren mit 10 ml 10%iger wässriger Natriumhydroxydlösung versetzt. Die organische Schicht wird abgetrennt und unter vermindertem Druck eingedampft. Man erhält die freie Base, welche nach Umkristallisation aus einem Gemisch von Methylenchlorid-Hexan bei 130° schmilzt.

*Tartrat*: Eine Lösung von 3,1 g der freien Base in 30 ml Aceton wird mit 2 g vorher getrockneter und gereinigter d-Weinsäure in aceton versetzt. Das ausgeschiedene Produkt wird mehrmals mit Diäthyläther und Aceton gewaschen. Der Rückstand wird aus Aceton umkristallisiert. Man erhält das N-Hexahydro-2(1H)-azocinyliden-N'-phenyl-4-morpholincarboximidamid-tartrat, welches bei 105° schmilzt.

*Sulfat*: Eine Lösung von 3 g der freien Base in 30 ml Methylenchlorid-Aceton wird unter Rühren mit 10 g Schwefelsäure in Methylenchlorid versetzt. Das Produkt scheidet sich in Form von weissen Kristallen aus. Nach Umkristallisation aus einem Gemisch von Isopropanol-Aceton erhält man das N-Hexahydro-2(1H)-azocinyliden-N'-phenyl-4-morpholin-carboximidamid-sulfat, welches bei 210° schmilzt.

*p-Toluolsulfonat*: Eine Lösung von 3,2 g der freien base in 30 ml Methylenchlorid-Aceton wird unter Rühren mit 1,8 g p-Toluolsulfonsäure versetzt. Das Produkt scheidet sich aus in Form von farblosen Kristallen. Nach Umkristallisation aus Isopropanol erhält man das N-Hexahydro-2(1H)-azocinyliden-N'-phenyl-4-morpholin-carboximidamid-p-toluolsulfonat, welches bei 198° schmilzt.

*Hydrochlorid*; Eine Lösung von 4 g der freien Base in 40 ml Isopropanol wird mit Chlorwasserstoffsäure in Isopropanol angesäuert. Das Lösungsmittel wird unter vermindertem Druck eingedampft und der Rückstand aus einem Gemisch von Isopropanol-Aceton umkristallisiert. Man erhält das N-Hexahydro-2(1H)-azocinyliden-N'-phenyl-4-morpholin-carboximidamid-hydrochlorid, welches bei 210° schmilzt.

*Methansulfonat*: Eine Lösung von 1,6 g der freien Base in Methylenchlorid wird mit 0,5 g Methansulfonsäure in Methylenchlorid versetzt. Das produkt scheidet sich in Form von farblosen Kristallen aus. Nach Umkristallisation aus einem Gemisch von Methanol-Aceton erhält man das N-Hexahydro-2(1H)-azocinyliden-N'-phenyl-4-morpholin-carboximidamid-methansulfonat, welches bei 212° schmilzt.

Der in dieser Synthese verwendete Ausgangsstoff wird wie folgt hergestellt: Eine Suspension von 29 g N-Phenyl-S-methyl-isothioharnstoff-hydrojodid in 90 ml Acetonitril wird mit 11 g Morpholin versetzt. Das Gemisch wird gerührt und 15 Stunden unter Rückfluss gekocht. Das Acetonitril wird unter vermindertem Druck abgedampft und der Rückstand aus einem Gemisch von Methylenchlorid-Essigsäureäthylester umkristallisiert. Man erhält das 1-(4-Morpholinyl)-N-phenyl-carboximidamid-hydrojodid, welches bei 182° schmilzt.

Beispiel 4

Eine suspension von 4 g 4,N-Diphenylpiperidin-carboximidamid-hydrojodid in 15 ml Acetonitril wird unter Rühren mit 2 g 3,4,5,6,7,8-Hexahydro-2-methoxyazocin versetzt. Das Gemisch wird auf dem Wasserbad 12 Stunden erhitzt und kräftig gerührt. Das Acetonitril wird unter vermindertem Druck abgedampft und der Rückstand aus einem Gemisch von Aceton-Essigsäureäthylester umkristallisiert. Man erhält das N-Hexahydro-2(1H)-azocinyliden-4,N'-diphenyl-piperidincarboximidamid-hydrojodid, welches bei 228—230° schmilzt.

Der Ausgangsstoff dieser Synthese wird wie folgt hergestellt: Eine Suspension von 10 g N-Phenyl-S-methyl-isothioharnstoff-hydrojodid in 30 ml Acetonitril wird unter Rühren mit 7 g 4-Phenylpiperidin versetzt. Das Gemisch wird 15 Stunden unter Rückfluss gekocht. Das Acetonitril wird unter vermindertem Druck abgedampft und der Rückstand aus einem Gemisch von Isopropanol-Essigsäureäthylester umkristallisiert. Man erhält das 4,N-Diphenylpiperidin-carboximidamid-hydrojodid, welches bei 151° schmilzt.

Beispiel 5

Eine Suspension von 3,5 g N-Phenyl-2,6-dimethyl-4-morpholin-carboximidamid-hydrojodid in 10 ml Acetonitril wird unter Rühren mit 1,5 g 3,4,5,6,7,8-Hexahydro-2-methoxyazocin versetzt. Das Gemisch wird auf dem Wasserbad 12 Stunden erhitzt und kräftig gerührt. Das Aceton wird unter vermindertem Druck abgedampft und der Rückstand aus einem Gemisch von Essigsäureäthylester-Aceton umkristallisiert. Man erhält das N-Hexahydro-2(1H)-azocinyliden-N'-phenyl-2,6-dimethyl-4- morpholincarboximidamid-hydrojodid, welches nach Umkristallisation aus einem Gemisch von Isopropanol-Essigsäureäthylester bei 235° schmilzt.

Die Ausgangsstoff dieser Synthese wird wie folgt hergestellt:

Eine Suspension von 15 g N-Phenyl-S-methyl-isothioharnstoff-hydrojodid in 50 ml Acetonitril wird mit 9 g 2,6-Dimethyl-morpholin versetzt. Das Gemisch wird unter Rühren 15 Stunden unter Rückfluss gekocht. Das Acetonitril wird unter vermindertem Druck abgedampft und der Rückstand aus einem Gemisch von Methylenchlorid-Essigsäureäthylester umkristallisiert. Man erhält das N-Phenyl-2,6-dimethyl-4-morpholin-carboximidamid-hydrojodid, welches bei 208—210° schmilzt.

Beispiel 6

Eine suspension von 8,2 g N' - (1 - Methyl - hexahydro - 2(1H) - azocinyliden) - N - phenyl - S - methyl - isothioharnstoff - hydrojodid in 30 ml Acetonitril wird unter Rühren mit 3 g Morpholin versetzt. Das Gemisch wird unter Rückfluss 36 Stunden in einem Oelbad gekocht. Das Acetonitril wird unter vermindertem Druck abgedampft und der Rückstand aus einem Gemisch von Essigsäureäthylester-Aceton umkristallisiert. Man erhält das N' - (1 - Methyl - hexahydro - 2(1H) - azocinyliden) - N - phenyl - 4 - morpholincarboximidamid - hydrojodid, welches nach Umkristallisation aus einem Gemisch von Isopropanol-Essigsäureäthylester bei 198° schmilzt.

Der Ausgangsstoff der obigen Synthese wird wie folgt hergestellt:

Eine Lösung von 14 g 1-Methyl-3,4,5,6,7,8-Hexahydroazocin-2-on in 50 ml Methylenchlorid wird unter Kühlen mit 40 g Triäthyloxoniumfluoroborat in 50 ml Methylenchlorid versetzt. Das Reaktionsgemisch wird 18 Stunden in einer Stickstoffatmosphäre gerührt. Dann leitet man 3 Stunden Ammoniak in das Gemisch ein. Das Reaktionsgemisch wird 30 Stunden bei Zimmertemperatur stehengelassen und das Lösungsmittel unter vermindertem Druck auf einem Wasserbad von 40° abgedampft. Das rohe 1-Methyl-2-imino-3,4,5,6,7,8-hexahydroazocin wird in 50 ml Acetonitril gelöst und unter Rühren bei Zimmertemperatur mit 12 g Phenylisothiocyanat in 30 ml Acetonitril versetzt. Das weisse kristalline Material, welches sich in fast 2 Stunden ausscheider, wird aus einem Gemisch von Essigsäureäthylester-Isopropanol umkristallisiert. Man erhält den N-Phenyl-N'-(1-methyl-hexahydro-2(1H)-azocinyliden)-thioharnstoff, welcher bei 123—125° schmilzt.

Eine Lösung von 14 g N-Phenyl-N'-(1-methyl-hexahydro-2(1H)-azocinyliden)-thioharnstoff in 50 ml Dioxan wird unter Rühren tropfenweise mit 10 g Methyljodid in 30 ml Dioxan versetzt. Das Gemisch wird 3 Stunden auf dem Wasserbad erhitzt. Das Dioxan wird unter vermindertem Druck abgedampft und der Rückstand aus einem Gemisch von Essigsäureäthylester-Aceton umkristallisiert. Man erhält das N'-(1-Methyl-hexahydro-2(1H)-azocinyliden-N-phenyl-S-methyl-isothioharnstoff-hydrojodid, welches bei 135° schmilzt.

Beispiel 7

Eine Suspension von 3,5 g N-(p-Fluorphenyl)-4-morpholin-carboximidamid-hydrojodid in 10 ml Acetonitril wird unter Rühren mit 2,5 g 3,4,5,6,7,8-Hexahydro-2-methoxyazocin versetzt und das Gemisch auf einem Wasserbad 12 Stunden unter kräftigem Rühren erhitzt. Das Acetonitril wird unter vermindertem Druck abgedampft und der Rückstand aus einem Gemisch von Isopropanol-Aceton umkristallisiert. Man erhält das N-Hexahydro-2(1H)-azocinyliden-N'-(p-fluorphenyl)-4-morpholincarboximidamid-hydrojodid, welches bei 245° schmilzt.

Der in dieser Synthese verwendete Ausgangsstoff wird wie folgt hergestellt: Eine Suspension von 7,5 g N-(p-Fluorphenyl)-S-methyl-isothioharnstoff-hydrochlorid in 30 ml Acetonitril wird mit 3 g Morpholin versetzt. Das Gemisch wird unter Rühren 15 Stunden unter Rückfluss gekocht. Das Acetonitril wird unter vermindertem Druck abgedampft und der Rückstand aus einem Gemisch von Aceton und Essigsäureäthylester umkristallisiert. Man erhält das N-(p-Fluorphenyl)-4-morpholincarboximidamid-hydrojodid, welches bei 210° schmilzt.

Beispiel 8

Eine Suspension von 2,5 g N-(m-Trifluoromethylphenyl)-4-morpholincarboximidamid-hydrojodid in 15 ml Acetonitril wird unter Rühren mit 1,5 g 3,4,5,6,7,8-Hexahydro-2-methoxyazocin versetzt. Das Gemisch wird auf dem Wasserbad gerührt und 12 Stunden unter Rückfluss gekocht. Das Acetonitril wird unter vermindertem Druck abgedampft und der Rückstand aus einem Gemisch von Diäthyläther-Essigsäureäthylester umkristallisiert. Man erhält das N-Hexahydro-2(1H)-azocinyliden-N'-(m-trifluoromethylphenyl)-4-morpholincarboximidamid-hydrojodid, welches in die freie Base und diese in das p-Toluolsulfonat umgewandelt wird. Nach Umkristallisation aus Aceton schmilzt das Produkt bei 202°.

Der Ausgangsstoff dieser Synthese wird wie folgt hergestellt:

Eine Lösung von 11 g N-(m-Trifluormethylphenyl)-thioharnstoff in 30 ml Dioxan wird mit 8 g Methyljodid in 20 ml Dioxan versetzt. Das Reaktionsgemisch wird auf dem Wasserbad 3 Stunden erhitzt.

Das Dioxan wird unter vermindertem Druck abgedampft und der Rückstand aus einem Gemisch von Isopropanol-Essigsäureäthylester umkristallisiert. Man erhält das N-(m-Trifluormethylphenyl)-S-methyl-isothioharnstoff-hydrojodid, welches bei 220° schmilzt.

Eine Suspension von 9 g N-(m-Trifluormethyl-phenyl)-S-methyl-isothioharnstoff-hydrojodid in 30 ml Acetonitril wird mit 3 g Morpholin versetzt. Das Gemisch wird unter Rühren 15 Stunden unter Rückfluss gekocht. Das Acetonitril wird unter vermindertem Druck abgedampft und der Rückstand aus einem Gemisch von Aceton-Essigsäureäthylester umkristallisiert. Man erhält das (m-Trifluormethylphenyl)-4-morpholincarboximidamid-hydrojodid, welches bei 220° schmilzt.

Beispiel 9

Eine Suspension von 3,5 g N-p-tolyl-4-morpholincarboximidamid-hydrojodid in 15 ml Acetonitril wird unter Rühren mit 2 g 3,4,5,6,7,8-Hexahydro-2-methoxyazocin versetzt. Das Gemisch wird auf dem Wasserbad 12 Stunden unter kräftigem Rühren erhitzt. Das Acetonitril wird unter vermindertem Druck abgedampft und der Rückstand aus einem Gemisch von Aceton-Essigsäureäthylester umkristallisiert. Man erhält das N-Hexahydro-2(1H)-azocinyliden-N'-p-tolyl-4-morpholincarboximidamid-hydrojodid. F.p. 240°.

Der Ausgantsstoff der obigen Synthese wird wie folgt hergestellt: Eine Suspension von 9 g N-p-Tolyl-S-methyl-isothioharnstoff-hydrojodid in 30 ml Acetonitril wird mit 3,5 g Morpholin versetzt. Das Gemisch wird unter Rühren 15 Stunden unter Rückfluss gekocht. Das Acetonitril wird unter vermindertem Druck abgedampft und der Rückstand aus einem Gemisch von Isopropanol und Essigsäureäthylester umkristallisiert. Man erhält das N-p-Tolyl-4-morpholincarboximidamid-hydrojodid, welches bei 218—220° schmilzt.

Beispiel 10

Eine Suspension von 3 g N-Phenyl-1-(4-carbäthoxypiperazin)-carboximidamid-hydrochlorid in 200 ml Acetonitril wird mit 2.8 g 1-Aza-2-methoxy-1-cycloocten versetzt und das Gemisch 12 Stunden unter Rückfluss gekocht. Das Lösungsmittel wird unter vermindertem Druck abgedampft und der Rückstand mit Essigsäureäthylester trituriert. Man erhält als farbloses festes Material das N - Hexahydro - 2(1H) - azocinyliden - N' - phenyl - 1 - (4 - carbäthoxypiperazin) - carboximidamid - hydrochlorid, welches nach Umkristallisation aus Acetonitril bei 248—249° schmilzt.

Der Ausgangsstoff obiger Synthese wird wie folgt hergestellt:

Eine Lösung von 8,7 g S-Methyl-phenylisothioharnstoff-hydrojodid in 30 ml Isopropanol wird mit 9,6 g N-Carbäthoxypiperazin in 15 ml Isopropanol versetzt und die Lösung 18 Stunden unter Rückfluss gekocht. Das Lösungsmittel wird unter vermindertem Druck eingedampft und der Rückstand mit Petroläther gewaschen. Man erhält das N-Phenyl-1-(4-carbäthoxy-piperazin)-carboximidamid-hydrojodid, welches nach Umkristallisation aus einem Gemisch von Isopropanol-Essigsäureäthylester bei 218—219° schmilzt.

Das obige Produkt wird mit gesättigter wässeriger Kaliumcarbonatlösung behandelt und das Gemisch mit Methylenchlorid extrahiert. Der Extrakt wird über wasserfreiem Natriumsulfat getrocknet, filtriert und das Lösungsmittel unter vermindertem Druck abgedampft. Der erhaltene Rückstand wird mit Chlorwasserstoff in Isopropanol behandelt. Man erhält das N-Phenyl-1-(4-carbäthoxy-piperazin)-carboximidamid-hydrochlorid, welches nach Umkristallisation aus einem Gemisch von Methanol und Essigsäureäthylester bei 251—252° schmilzt.

Beispiel 11

Ein Gemisch von 2,2 g N-(p-Methoxyphenyl)-1-4-carbäthoxy-piperazin)-carboximidamid-hydrojodid und 1.4 g 3,4,5,6,7,8-Hexahydro-2-methoxyazorin wird in 7 ml Acetonitril gelöst und die Lösung 10 Stunden unter Rückfluss gekocht. Die klare Lösung wird abgekühlt und der erhaltene Niederschlag abfiltriert. Man erhält das N - Hexahydro - 2(1H) - azocinyliden - N' - (p - methoxyphenyl) - 1 - (4 - carbäthoxy - piperazin) - carboximidamid - hydrojodid, welches nach Umkristallisation aus einem Gemisch von Acetonitril-Essigsäureäthylester bei 241—242° schmilzt.

Der Ausgangsstoff für das obige Produkt wird wie folgt erhalten:

Ein Gemisch von 10 g p-Anisidin-hydrochlorid und 12,25 g Kaliumthiocyanat wird in 100 ml Aethanol 16 Stunden erhitzt. Das Reaktionsgemisch wird gekühlt und filtriert. Der Rückstand wird mit Wasser gewaschen und aus Methanol umkristallisiert. Man erhält ein festes, bei 226° schmelzendes Material, welches aus p-Methoxyphenylthioharnstoff besteht. Es werden 5,3 g der letztgenannten Verbindung mit 10 ml Methyljodid in 20 ml Aceton 8 Stunde auf 50° erhitzt. Das Reaktionsgemisch wird gekühlt, der Niederschlag abfiltriert und mit Aceton gewaschen. Man erhält das S-Methyl-N-(p-methoxyphenyl)-isothioharnstoff-hydrojodid, welches bei 165—166° schmilzt.

Ein Gemisch von 6,4 g S-Methyl-N-(p-methoxyphenyl)-isothioharnstoff-hydrojodid und 6,2 g N-Carbäthoxy-piperazin in 50 ml Acetonitril wird 10 Stunden unter Rückfluss gekocht. Das Lösungsmittel wird unter vermindertem Druck eingedampft und der Rückstand mit Essigsäureäthylester trituriert. Man erhält das N-(p-Methoxyphenyl)-1-(4-carbäthoxypiperazin)-carboximidamid-hydrojodid, welches nach Umkristallisation aus Acetonitril bei 156—157°Schmilzt.

Beispiel 12

Eine Lösung von 2,4 g N-Phenyl-1-(4-methyl-piperazin)-carboximidamid-hydrojodid in 6 ml Acetonitril wird unter Rühren mit 2,3 g 3,4,5,6,7,8-Hexahydro-2-methoxyazocin versetzt und 12 Stunden unter Rückfluss gekocht. Das Reaktionsgemisch wird gekühlt und mit 10 ml Essigsäureäthylester verdünnt. Es

entsteht ein amorphes festes Material, welches nach Triturieren mit Aceton kristallin wird. Man erhält das N - Hexahydro - 2(1H) - azocinyliden - N' - phenyl - 1 - (4 - methylpiperazin) - carboximidamid - hydrojodid, welches nach Umkristallisation aus Acetonitril bei 222—223° schmilzt.

Der für die Synthese des obigen Produkts verwendete Ausgangsstoff wird wie folgt hergestellt:

Ein Gemisch von 10 g S-Methyl-phenylisothioharnstoffhydrojodid und 7,6 g N-Methylpiperazin wird in 50 ml Isopropanol gelöst und die Lösung 10 Stunden unter Rückfluss gekocht. Das Lösungsmittel wird unter vermindertem Druck eingedampft. Der Rückstand wird mit Petroläther behandelt. Man erhält das 1-(4-methylpiperazin)-N-phenyl-carboximidamid-hydrojodid, welches nach Umkristallisation aus einem Gemisch von Acetonitril und Essigsäureäthylester bei 231—232° schmilzt.

**Patentansprüche für die Vertragsstaaten: BE CH DE FR GB IT LI LU NL SE**

1. Neue Guanidinderivate der Formel I

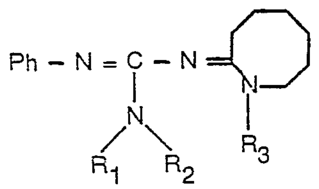

(I)

worin Ph einen gegebenenfalls durch Niederalkyl, Hydroxy, Mercapto, Niederalkoxy, Niederalkenyloxy Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander Niederalkyl oder Cycloalkyl mit höchstens 10 Kohlenstoffatomen oder beide zusammengenommen einen gegebenenfalls durch Niederalkyl oder Phenyl substituierten bivalenten Kohlenwasserstoffrest aliphatischen Charakters, mit 4—7 Kohlenstoffatomen in der Kette in welchen die Kohlenstoffatome der Kette durch Sauerstoff, Schwefel oder Stickstoff unterbrochen sein können, $R_3$ Wasserstoff oder Niederalkyl bedeuten, ihre tautomeren Verbindungen und Salze, wobei die mit "nieder" bezeichneten Reste höchstens 7 Kohlenstoffatome aufweisen.

2. Verbindungen der Formel I, in denen Ph einen gegebenenfalls durch Niederalkyl, Hydroxy, Mercapto, Niederalkoxy, Niederalkenyloxy, Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander einen Methyl- oder Aethyl-, Cyclopentyl-, Cyclohexyl- oder Cycloheptylrest oder beide zusammengenommen einen gegebenenfalls durch Niederalkyl oder Phenyl substituierten bivalenten Kohlenwasserstoffrest aliphatischen Charakters mit 4—7 Kohlenstoffatomen in der Kette, in welchem die Kohlenstoffatome der Kette durch ein Sauerstoff, Schwefel oder Stickstoff unterbrochen sein können, $R_3$ Wasserstoff oder Niederalkyl bedeuten, ihre tautomeren Verbindungen und Salze, wobei die mit "nieder" bezeichneten Reste höchstens 7 Kohlenstoffatome aufweisen.

3. Verbindungen der Formel I, in denen Ph einen gegebenenfalls durch Niederalkyl, Niederalkoxy, Halogen oder Trifluormethyl substituiertes Phenyl, $R_1$ und $R_2$ beide zusammengenommen eine gegebenenfalls durch Niederalkyl oder Phenyl substituiert Niederalkylenkette, in welcher die Kohlenstoffatome der Kette Durch Sauerstoff, Schwefel oder gegebenenfalls durch Niederalkyl, Phenyl, Benzyl, Phenyläthyl oder Alkoxycarbonyl substituierten Stickstoff unterbrochen sein können, und $R_3$ Wasserstoff oder Niederalkyl bedeuten, ihre tautomeren Verbindungen und Salze, wobei die mit "nieder" bezeichneten Reste höchstens 7 Kohlenstoffatome aufweisen.

4. Verbindungen der Formel I, in denen Ph einen gegebenenfalls durch Niederalkyl, Niederalkoxy, Halogen oder Trifluormethyl substituiertes Phenyl bedeutet, die Gruppe $—NR_1R_2$ Pyrrolidino, 2,5-Dimethylpyrrolidino, Piperidino, 2-Methyl-, 4-Methyl- oder 4-Phenylpiperidino, Hexahydroazepino, Morpholino, 2,6-Dimethylmorpholino, Thiomorpholino, 2,6-Dimethylthiomorpholino, Piperazino, N-Methyl-, N-Phenyl-, N-Benzyl, N-Methoxy-, N-Aethoxycarbonylpiperazin sein kann, und $R_3$ Wasserstoff oder auch Niederalkyl bedeutet, ihre tautomeren Verbindungen und Salze, wobei die mit "nieder" bezeichneten Reste höchstens 7 Kohlenstoffatome aufweisen.

5. Verbindungen der Formel I, in denen Ph ein gegebenenfalls durch Niederalkyl, wie z.B. Methyl oder Aethyl, Niederalkoxy, wie z.B. Methoxy oder Aethoxy, Halogen, wie z.B. Chlor oder Brom oder Trifluormethyl substituiertes Phenyl bedeutet, die Gruppe $—NR_1R_2$ Pyrrolidino, Piperidino, 4-Methyl- oder 4-Phenylpiperidino, Morpholino, 2,6-Dimethylmorpholino, Piperazino, N-Methyl, N-Methoxycarbonylpiperazin sein kann, und $R_3$ Wasserstoff oder auch Methyl oder Aethyl bedeutet, ihre tautomeren Verbindungen und Salze.

6. N-Hexahydro-2-(1H)-azocinyliden-N'-phenyl-1-pyrrolidin-carboxyimidamid.

7. N-Hexahydro-2(1H)-azocinyliden-N'-phenyl-1-piperidin-carboximidamid.

8. N-Hexahydro-2(1H)-azocinyliden-N'-phenyl-4-morpholincarboximidamid.

9. N-Hexahydro-2(1H)-azocinyliden-4,N'-diphenylpiperidincarboximidamid.

10. N-Hexahydro-2(1H)-azocinyliden-N'-phenyl-2,6-dimethyl-4-morpholincarboximidamid.

11. N'-(1-Methyl-hexahydro-2(1H)-azocinyliden)-N-phenyl-4-morpholincarboximidamid.

12. N-Hexahydro-2(1H)-azocinyliden-N'-(p-fluorphenyl)-4-morpholincarboximidamid.

13. N-Hexahydro-2(1H)-azocinyliden-N'-(m-trifluoromethylphenyl)-4-morpholincarboximidamid.

14. N-Hexahydro-2(1H)-azocinyliden-N'-p-tolyl-4-morpholincarboximidamid.

15. N-Hexahydro-2(1H)-azocinyliden-N'-phenyl-1-(4-carbäthoxy-piperazin)-carboximidamid.

16. N-Hexahydro-2(1H)-azocinyliden-N'-(p-methoxyphenyl)-1-(4-carbäthoxy-piperazin)-carboximidamid.

17. N-Hexahydro-2(1H)-azocinyliden-N'-phenyl-(4-methylpiperazin)-carboximidamid.

18. Tautomere Verbindungen der Ansprüche 6—17.

19. Salze von Verbindungen der Ansprüche 6—18.

20. Pharmazeutisch verwendbare, ncht toxische Salze von Verbindungen der Ansprüche 6—18.

21. Hypoglykämisch wirksame Verbindungen der Formel I und entsprechende tautomere Verbindungen gemäss Anspruch 1 und Salze von solchen Verbindungen.

22. Pharmazeutische Präparate enthaltend eine der Verbindungen der Ansprüche 1—17 und 20.

23. Die Verbindungen der Ansprüche 1—17 und 20, zur Verwendung als Antidiabetica.

24. Verwendung der Verbindungen der Ansprüche 1 bis 17 und 20 zur Herstellung von pharmazeutischen Präparaten.

25. Verfahren zur Herstellung von neuen Guanidinderivaten der Formel I

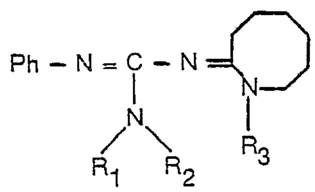

(I)

worin Ph einen gegebenenfalls durch Niederalkyl, Hydroxy, Mercapto, Niederalkoxy, Niederalkenyloxy, Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander Niederalkyl oder Cycloalkyl mit höchstens 10 Kohlenstoffatomen oder beide zusammengenommen einen gegebenenfalls durch Niederalkyl oder Phenyl substituierten bivalenten Kohlenwasserstoffrest aliphatischen Charakters, mit 4—7 Kohlenstoffatomen in der Kette in welchen die Kohlenstoffatome der Kette durch Sauerstoff, Schwefel oder Stickstoff unterbrochen sein können, $R_3$ Wasserstoff oder Niederalkyl bedeuten, ihre tautomeren Verbindungen und Salze, wobei die mit "nieder" bezeichneten Reste höchstens 7 Kohlenstoffatome aufweisen, dadurch gekennzeichnet, dass man

a) eine Verbindung der Formel II

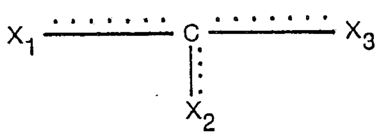

(II)

worin $X_1$ die Gruppe Ph—N=, in der Ph einen gegebenenfalls substituierten Phenylrest bedeutet, oder eine abspaltbare Gruppe, $X_2$ die Gruppe —$NR_1R_2$, worin $R_1$ und $R_2$ die unter der Formel I angegebene Bedeutung haben, oder eine abspaltbare Gruppe und $X_3$ die Gruppe

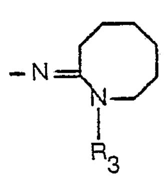

,

worin $R_3$ die unter der Formel I angegebene Bedeutung hat, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ oder $X_3$ mit dem Kohlenstoffatom durch eine Doppelbindung gebunden ist, mit einem Amin oder Imin umsetzt, welches der fehlenden Amino- oder Iminogruppe entspricht, die unter $X_1$, $X_2$ oder $X_3$ definiert werden, um die abspaltbare Gruppe zu ersetzen, oder

b) eine Verbindung der Formel IV

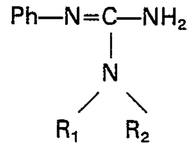

(IV),

worin Ph, $R_1$ und $R_2$ die unter der Formel I angegebenen Bedeutungen haben, mit einer Verbindung der allgemeinen Formel V

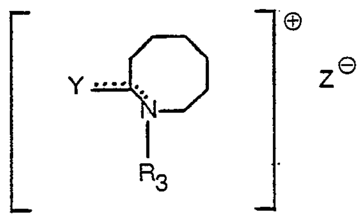

(V) ,

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, Niederalkansulfonatanion oder ein Halogenid bedeutet, wobei, wenn Y die Bedeutung von zwei Niederalkoxygruppen am gleich C-Atom hat, Z als Anion enthällt, oder, wenn $R_3$ Wasserstoff bedeutet, die tautomere Form als freie Base vorliegt, umsetzt, oder

c) Verbindungen der Formel I, in der $R_1$ die oben definierte Bedeutung hat und $R_2$ und/oder $R_3$ Wasserstoff bedeuten, durch Umsetzung mit einem reaktionsfähigen Ester eines Niederalkanols oder gegebenenfalls Cycloalkanols in Verbindungen der Formel I unwandelt, in denen $R_2$ und/oder $R_3$ im Rahmen der vorstehenden Definitionen für $R_2$ und $R_3$ verschieden von Wasserstoff sind, oder

d) in Verbindungen der allgemeinen Formel VI

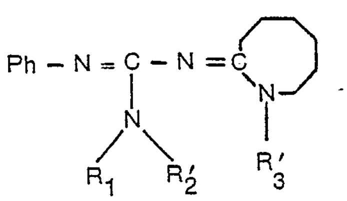

(VI) ,

worin $R_1$ und Ph die unter der Formel I angegebenen Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

**Patentansprüche für den Vertragsstaat: AT**

1. Verfahren zur Herstellung von neuen Guanidinderivaten der Formel I

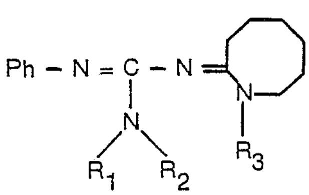

(I)

worin Ph einen gegebenenfalls durch Niederalkyl, Hydroxy, Mercapto, Niederalkoxy, Niederalkenyloxy Niederalkylendioxy, Niederalkylthio, Halogen, Trifluoromethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander Niederalkyl oder Cycloalkyl mit höchstens 10 Kohlenstoffatomen oder beide zusammengenommen einen gegebenenfalls durch Niederalkyl oder Phenyl substituierten bivalenten Kohlenwasserstoffrest aliphatischen Charakters, in welchem die Kohlenstoffatome der Kette durch Sauerstoff, Schwefel oder Stickstoff unterbrochen sein können, $R_3$ Wasserstoff oder Niederalkyl bedeuten, ihre tautomeren Verbindungen und Salze, wobei die mit "nieder" bezeichneten Reste höchstens 7 Kohlenstoffatome aufweisen, dadurch gekennzeichnet, dass man

a) eine Verbindung der Formel II

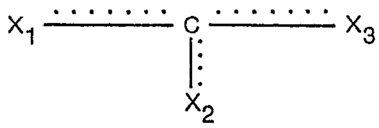

(II)

worin $X_1$ die Gruppe Ph—N=, in der Ph einen gegebenenfalls substituierten Phenylrest bedeutet, oder eine abspaltbare Gruppe, $X_2$ die Gruppe —$NR_1R_2$, worin $R_1$ und $R_2$ die unter der Formel I angegebene Bedeutung haben, oder eine abspaltbare Gruppe und $X_3$ die Gruppe

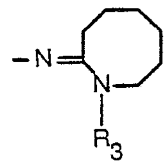

,

worin $R_3$ die unter der Formel I angegebene Bedeutung hat, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ oder $X_3$ mit dem Kohlenstoffatom durch eine Doppelbildung gebunden ist, mit einem Amin oder Imin umsetzt, welches der fehlenden Amino- oder Iminogruppe entspricht, die unter $X_1$, $X_2$ oder $X_3$ definiert werden, um die abspaltbare Gruppe zu ersetzen, oder

b) eine Verbindung der Formel IV

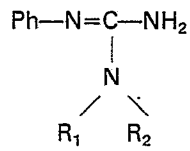

(IV),

worin Ph, $R_1$ und $R_2$ die unter der Formel I angegebenen Bedeutungen haben, mit einer Verbindung der allgemeinen Formel V

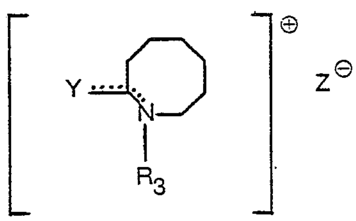

(V),

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, Niederalkansulfonatanion oder ein Halogenid bedeutet, wobei, wenn Y die Bedeutung von zwei Niederalkoxygruppen am gleich C-Atom hat, Z als Anion entfällt, oder, wenn $R_3$ Wasserstoff bedeutet, die tautomere Form als freie Base vorliegt, umsetzt, oder

c) Verbindung der Formel I, in der $R_1$ die oben definierte Bedeutung hat und $R_2$ und/oder $R_3$ Wasserstoff bedeuten, durch Umsetzung mit einem reaktionsfähigen Ester eines Niederalkanols oder gegebenenfalls Cycloalkanols in Verbindungen der Formel I unwandelt, in denen $R_2$ und/oder $R_3$ im Rahmen der vorstehenden Definitionen für $R_2$ und $R_3$ verschieden von Wasserstoff sind, oder

d) in Verbindungen der allgemeinen Formel VI

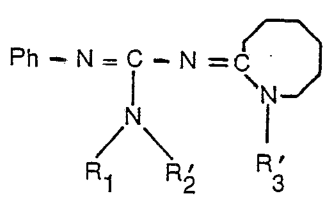

(VI),

worin $R_1$ und Ph die unter der Formel I angegebenen Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

2. Verfahren nach Patentanspruch 1, dadurch gekennzeichnet, dass man eine Verbindung der Formel IIa

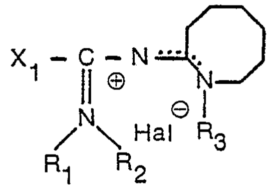

(IIa),

worin $X_1$ eine abspaltbare Gruppe bedeutet und $R_1$, $R_2$ und $R_3$ die unter Formel I angegebenen Bedeutungen haben, mit einem gegebenenfalls substituierten Anilin der Formel Ph—$NH_2$ umsetzt.

3. Verfahren nach Patentanspruch 1, dadurch gekennzeichnet, dass man eine Verbindung der Formel IIb

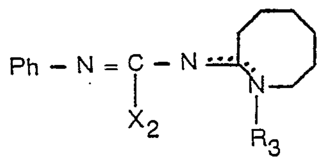

(IIb),

worin $X_2$ Eine abspaltbare Gruppe bedeutet und Ph und $R_3$ unter der Formel I angegebenen Bedeutungen haben, mit einem Amin der Formel $HNR_1R_2$ umsetzt.

4. Verfahren nach Patentanspruch 1, dadurch gekennzeichnet, dass man eine Verbindung der Formel IIc

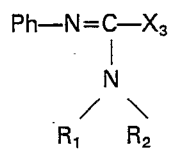

(IIc),

worin $X_3$ eine abspaltbare Gruppe bedeutet und Ph, $R_1$ und $R_2$ die unter Formel I angegebenen Bedeutungen haben mit einer Iminoverbindung der Formel III

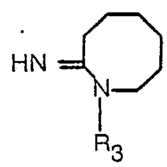

(III)

umsetzt.

5. Verfahren nach Patentansprüche 1—4, dadurch gekennzeichnet, dass ein abspaltbarer Rest in den Verbindungen der Formel II, IIa, IIb und IIc ein Niederalkylthio, Niederalkoxy oder ein Halogen bedeutet, wobei die mit "nieder" bezeichneten Reste höchstens 7 Kohlenstoffatome aufweisen.

6. Verfahren nach Patentanspruch 1—5, dadurch gekennzeichnet, dass ein abspaltbarer Rest in den Verbindungen der Formeln II, IIa, IIb und ein Methylthio, Methoxy oder ein Chlor oder Bromatom bedeutet.

7. Verfahren nach Patentanspruch 1, dadurch gekennzeichnet, dass man neue Guanidinderivate der Formel I

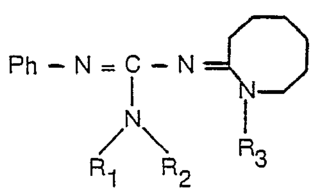

(I)

worin Ph einen gegebenenfalls durch Niederalkyl, Hydroxy, Mercapto, Niederalkoxy, Niederalkenyloxy Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander ein Niederalkyl oder Cycloalkyl mit höchstens 10 Kohlenstoffatomen oder beide zusammengenommen einen gegebenenfalls durch Niederalkyl oder Phenyl substituierten bivalenten Kohlenwasserstoffrest aliphatischen Charakters, in welchem die Kohlenstoffatome der Kette durch Sauerstoff, Schwefel oder Stickstoff unterbrochen sein können, $R_3$ Wasserstoff oder Niederalkyl bedeuten, ihre tautomeren Verhindungen und Salze wobei die mit "nieder" bezeichneten Reste höchstens 7 Kohlenstoffatome aufweisen, herstellt.

8. Verfahren nach Patentanspruch 1, dadurch gekennzeichnet, dass man Verbindungen der Formel I, in denen Ph einen gegebenenfalls durch Niederalkyl, Hydroxy, Mercapto, Niederalkoxy, Niederalkenyloxy, Niederalkylendioxy, Niederalkylthio, Halogen, Trifluormethyl, Nitro, Amino, Niederalkylamino, Diniederalkylamino, Sulfamyl, Niederalkylsulfamyl, Diniederalkylsulfamyl, Carboxy oder Niederalkoxycarbonyl substituierten Phenylrest, $R_1$ und $R_2$ unabhängig voneinander einen Methyl- oder Aethyl-, Cyclopentyl-, Cyclohexyl- oder Cycloheptylrest oder beide zusammengenommen einen gegebenenfalls durch Niederalkyl oder Phenyl substituierten bivalenten Kohlenwasserstoffrest aliphatischen Charakters mit 4—7 Kohlenstoffatomen in der Kette, in welchem die Kohlenstoffatome der Kette durch ein Sauerstoff, Schwefel oder Stickstoff unterbrocken sein können, $R_3$ Wasserstoff oder Niederalkyl bedeuten, ihre tautomeren Verbindungen und Salze, wobei die mit "nieder" bezeichneten Reste höchstens 7 Kohlenstoffatome aufweisen, herstellt.

9. Verfahren nach Patentanspruch 1, dadurch gekennzeichnet, dass man N-Hexahydro-2(1H)-azocinyliden-N'-phenyl-1-pyrrolidincarboximidamid herstellt.

10. Verfahren nach Patentanspruch 1, dadurch gekennzeichnet, dass man N-Hexahydro-2(1H)-azocinyliden-N'-phenyl-1-piperidincarboximidamid herstellt.

11. Verfahren nach Patentanspruch 1, dadurch gekennzeichnet, dass man N-Hexahydro-2(1H)-azocinyliden-N'- phenyl-4-morpholincarboximidamid herstellt.

12. Verfahren nach Patentanspruch 1, dadurch gekennzeichnet, dass man N-Hexahydro-2(1H)-azocinyliden-N'-phenyl-2,6-dimethyl-4-morpholincarboximidamid herstellt.

**Claims for the Contracting States: BE CH DE FR GB IT LI LU NL SE**

1. A novel guanidine derivative of the formula I

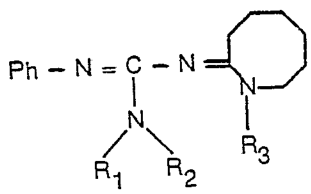

(I)

wherein Ph is a phenyl group which is unsubstituted or substituted by lower alkyl, hydroxy, mercapto, lower alkoxy, lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is lower alkyl or cycloalkyl containing not more than 10 carbon atoms, or both taken together are a bivalent hydrocarbon radical of aliphatic character containing 4 to 7 carbon atoms in the chain, which radical is unsubstituted or substituted by lower alkyl or phenyl, and in which radical the carbon atoms of the chain may be interrupted by oxygen, sulfur or nitrogen, and $R_3$ is hydrogen or lower alkyl, or a tautomeric compound or a salt thereof, with the radicals qualified by the term "lower" containing not more than 7 carbon atoms.

2. A compound of the formula I, wherein Ph is a phenyl group which is unsubstituted or substituted by lower alkyl, hydroxy, mercapto, lower alkoxy, lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is a methyl or ethyl, cyclopentyl, cyclohexyl or cycloheptyl radical, or both taken together are a bivalent hydrocarbon radical of aliphatic character containing 4 to 7 carbon atoms in the chain, which radical is unsubstituted or substituted by lower alkyl or phenyl, and in which radical the carbon atoms of the chain may be interrupted by an oxygen, sulfur or nitrogen atom, and $R_3$ is hydrogen or lower alkyl, or a tautomeric compound or a salt thereof, with the radicals qualified by the term "lower" containing not more than 7 carbon atoms.

3. A compound of the formula I, wherein Ph is a phenyl group which is unsubstituted or substituted by lower alkyl, lower alkoxy, halogen or trifluoromethyl, and $R_1$ and $R_2$ both taken together are a lower alkylene chain which is unsubstituted or substituted by lower alkyl or phenyl, and in which the carbon atoms of the chain may be interrupted by oxygen or sulfur, or by nitrogen which is unsubstituted or substituted by lower alkyl, phenyl, benzyl, phenylethyl or alkoxycarbonyl, and $R_3$ is hydrogen or lower alkyl, or a tautomeric compound or a salt thereof, with the radicals qualified by the term "lower" containing not more than 7 carbon atoms.

4. A compound of the formula I, wherein Ph is a phenyl group which is unsubstituted or substituted by lower alkyl, lower alkoxy, halogen or trifluoromethyl, the group $—NR_1R_2$ may be pyrrolidino, 2,5-dimethylpyrrolidino, piperidino, 2-methyl-, 4-methyl- or 4-phenylpiperidino, hexahydroazepino, morpholino, 2,6-dimethylmorpholino, thiomorpholino, 2,6-dimethylthiomorpholino, piperazino, N-methyl-, N-phenyl-, N-benzyl-, N-methoxy-, N-ethoxycarbonylpiperazine, and $R_3$ is hydrogen or lower alkyl, or a tautomeric compound or a salt thereof, with the radicals qualified by the term "lower" containing not more than 7 carbon atoms.

5. A compound of the formula I, wherein Ph is a phenyl group which is unsubstituted or substituted by lower alkyl, such as methyl or ethyl, lower alkoxy, such as methoxy or ethoxy, halogen, such as chlorine or bromine, or trifluoromethyl, the group $—NR_1R_2$ may be pyrrolidino, piperidino, 4-methyl- or 4-phenylpiperidino, morpholino, 2,6-dimethylmorpholino, piperazino, N-methyl- or N-methoxycarbonylpiperazine, and $R_3$ is hydrogen or alternatively methyl or ethyl, or a tautomeric compound or a salt thereof.

6. N-Hexahydro-2(1H)-azocinylidene-N'-phenyl-1-pyrrolidinecarboximide-amide.
7. N-Hexahydro-2(1H)-azocinylidene-N'-phenyl-1-piperidinecarboximide-amide.
8. N-Hexahydro-2(1H)-azocinylidene-N'-phenyl-4-morpholinecarboximide-amide.
9. N-Hexahydro-2(1H)-azocinylidene-4,N'-diphenylpiperidinecarboximide-amide.
10. N-Hexahydro-2(1H)-azocinylidene-N'-phenyl-2,6-dimethyl-4-morpholinecarboximide-amide.
11. N'-(1-Methylhexahydro-2(1H)-azocinylidene)-N-phenyl-4-morpholinecarboximide-amide.
12. N-Hexahydro-2(1H)-azocinylidene-N'-(p-fluorophenyl)-4-morpholinecarboximide-amide.
13. N-Hexahydro-2(1H)-azocinylidene-N'-(m-trifluoromethylphenyl)-4-morpholinecarboximide-amide.
14. N-Hexahydro-2(1H)-azocinylidene-N'-p-tolyl-4-morpholinecarboximide-amide.
15. N-Hexahydro-2(1H)-azocinylidene-N'-phenyl-1-(4-carbethoxypiperazine)carboximide-amide.
16. N-Hexahydro-2(1H)-azocinylidene-N'-(p-methoxyphenyl)-1-(4-carbethoxypiperazine)carboximide-amide.
17. N-Hexahydro-2(1H)-azocinylidene-N'-phenyl-(4-methylpiperazine)carboximide-amide.
18. A tautomeric compound as claimed in any one of claims 6 to 17.
19. A salt of a compound as claimed in any one of claims 6 to 18.
20. A pharmaceutically acceptable nontoxic salt of a compound as claimed in any one of claims 6 to 18.
21. A hypoglycaemically effective compound of the formula I or a corresponding tautomeric compound according to claim 1, or a salt thereof.
22. A pharmaceutical composition containing one of the compounds as claimed in any one of claims 1 to 17 and 20.
23. Use of a compound as claimed in any one of claims 1 to 17 and 20 as antidiabetic agent.
24. Use of a compound as claimed in any one of claims 1 to 17 and 20 for the preparation of a pharmaceutical composition.
25. A process for the preparation of a novel guanidine derivative of the formula I

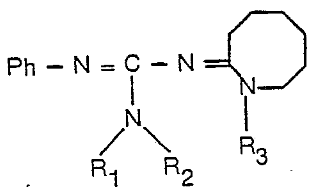

(I)

wherein Ph is a phenyl group which is unsubstituted or substituted by lower alkyl, hydroxy, mercapto, lower alkoxy, lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is lower alkyl or cycloalkyl containing not more than 10 carbon atoms, or both taken together are a bivalent hydrocarbon radical of aliphatic character containing 4 to 7 carbon atoms in the chain, which radical is unsubstituted or substituted by lower alkyl or phenyl, and in which radical the carbon atoms of the chain may be interrupted by oxygen, sulfur or nitrogen, and $R_3$ is hydrogen or lower alkyl, or a tautomeric compound or a salt thereof, with the radicals qualified by the term "lower" containing not more than 7 carbon atoms, which process comprises

a) reacting a compound of the formula II

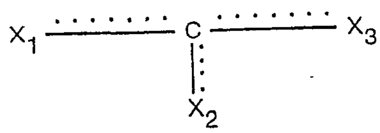

(II)

wherein $X_1$ is the group Ph—N=, in which Ph is a substituted or unsubstituted phenyl group, or a removable group, $X_2$ is the group $—NR_1R_2$, wherein $R_1$ and $R_2$ are as defined for formula I, or a removable group, and $X_3$ is the group

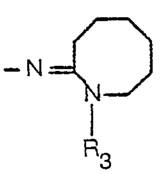

,

wherein $R_3$ is as defined for formula I, or a removable group, with the proviso that only one of the

substituents $X_1$, $X_2$ or $X_3$ can be a removable group and wherein one of the groups $X_1$, $X_2$ or $X_3$ is attached through a double bond to the carbon atom, with an amine or imine corresponding to the eliminated amino or imino group falling within the definition of $X_1$, $X_2$ or $X_3$, in order to replace the removable group; or

b) reacting a compound of the formula IV

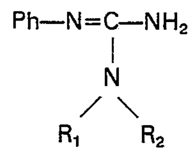

(IV)

wherein Ph, $R_1$ and $R_2$ are as defined for formula I, with a compound of the general formula V

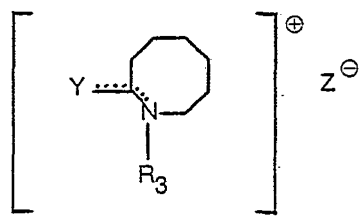

(V) ,

wherein Y is lower alkoxy, lower alkylthio, halogen, or Y is two lower alkoxy groups which are located on the same C atom, and Z is a tetrafluoroborate anion, a fluorosulfonate anion, a lower alkylsulfate anion, a lower alkanesulfonate anion, or a halide, with the proviso that, if Y is two lower alkoxy groups on the same C atom, Z as anion is not present, or, if $R_3$ is hydrogen, the tautomeric form is obtained as a free base; or

c) converting a compound of the formula I, in which $R_1$ is as defined above, and $R_2$ and/or $R_3$ are hydrogen, by reaction with a reactive ester of a lower alkanol or optionally of a cycloalkanol, into a compound of the formula I, in which $R_2$ and/or $R_3$, within the scope of the definitions for $R_2$ and $R_3$ given in the foregoing, are other than hydrogen; or

d) in a compound of the general formula VI

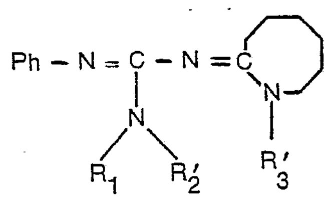

(VI) ,

wherein $R_1$ and Ph are as defined for formula I, and one of the substituents $R_2'$ and $R_3'$ has the meaning of $R_2$ or $R_3$, and the other is an amino protecting group, or both $R_2'$ and $R_3'$ are an amino protecting group, removing said group; and, if desired, carrying out additional process steps; and/or, if desired, converting a resultant compound of the formula I into a salt; and/or, if desired, converting a salt of a compound of the formula I into a free base.

**Claims for the Contracting State: AT**

1. A process for the preparation of a novel guanidine derivative of the formula I

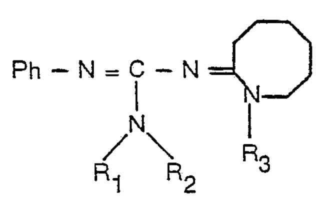

(I)

wherein Ph is a phenyl group which is unsubstituted or substituted by lower alkyl, hydroxy, mercapto, lower alkoxy, lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is lower alkyl or cycloalkyl containing not more than 10 carbon atoms, or both taken together are a bivalent hydrocarbon radical of aliphatic character, which radical is unsubstituted or substituted by lower alkyl or phenyl, and in which radical the carbon atoms of the chain may be interrupted by oxygen, sulfur or nitrogen, and $R_3$ is hydrogen or lower alkyl, or a tautomeric compound or a salt thereof, with the radicals qualified by the term "lower" containing not more than 7 carbon atoms, which process comprises

a) reacting a compound of the formula II

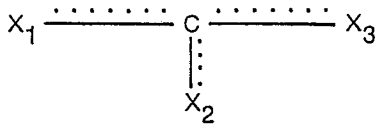

(II)

wherein $X_1$ is the group Ph—N=, in which Ph is a substituted or unsubstituted phenyl group, or a removable group, $X_2$ is the group $—NR_1R_2$, wherein $R_1$ and $R_2$ are as defined for formula I, or a removable group, and $X_3$ is the group

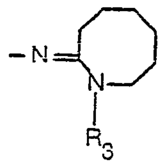

,

wherein $R_3$ is as defined for formula I, or a removable group, with the proviso that only one of the substituents $X_1$, $X_2$ or $X_3$ can be a removable group and wherein one of the groups $X_1$, $X_2$ or $X_3$ is attached through a double bond to the carbon atom, with an amine or imine corresponding to the eliminated amino or imino group falling within the definition of $X_1$, $X_2$ or $X_3$, in order to replace the removable group; or

b) reacting a compound of the formula IV

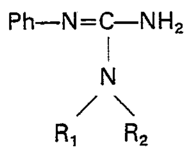

(IV)

wherein Ph, $R_1$ and $R_2$ are as defined for formula I, with a compound of the general formula V

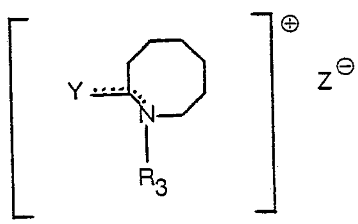

(V) ,

wherein Y is lower alkoxy, lower alkylthio, halogen, or Y is two lower alkoxy groups which are located on the same C atom, and Z is a tetrafluoroborate anion, a fluorosulfonate anion, a lower alkylsulfate anion, a lower alkanesulfonate anion, or a halide, with the proviso that, if Y is two lower alkoxy groups on the same C atom, Z as anion is not present, or, if $R_3$ is hydrogen, the tautomeric form is obtained as a free base; or

c) converting a compound of the formula I, in which $R_1$ is as defined above, and $R_2$ and/or $R_3$ are hydrogen, by reaction with a reactive ester of a lower alkanol or optionally of a cycloalkanol, into a compound of the formula I, in which $R_2$ and/or $R_3$, within the scope of the definitions for $R_2$ and $R_3$ given in the foregoing, are other than hydrogen; or

d) in a compound of the general formula VI

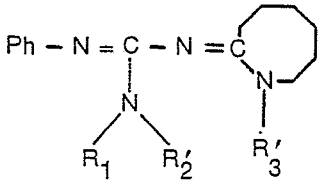

(VI) ,

wherein $R_1$ and Ph are as defined for formula I, and one of the substituents $R_2'$ and $R_3'$ has the meaning of $R_2$ or $R_3$, and the other is an amino protecting group, or both $R_2'$ and $R_3'$ are an amino protecting group, removing said group; and, if desired, carrying out additional process steps; and/or, if desired, converting a resultant compound of the formula I into a salt; and/or, if desired, converting a salt of a compound of the formula I into a free base.

2. A process according to claim 1, which comprises reacting a compound of the formula IIa

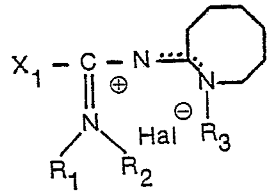

(IIa) ,

wherein $X_1$ is a removable group, and $R_1$, $R_2$ and $R_3$ are as defined for formula I, with an unsubstituted or substituted aniline of the formula Ph—$NH_2$.

3. A process according to claim 1, which comprises reacting a compound of the formula IIb

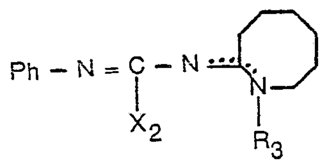

(IIb) ,

wherein $X_2$ is a removable group, and Ph and $R_3$ are as defined for formula I, with an amine of the formula $HNR_1R_2$.

4. A process according to claim 1, which comprises reacting a compound of the formula IIc

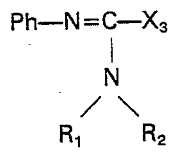

(IIc)

wherein $X_3$ is a removable group, and Ph, $R_1$ and $R_2$ are as defined for formula I, with an imino group of the formula III

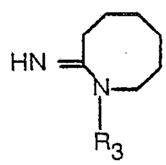

(III)

5. A process according to any one of claims 1 to 4, wherein a removable group in a compound of the formula II, IIa, IIb or IIc is a lower alkylthio group, a lower alkoxy group or a halogen atom, with the groups qualified by the term "lower" containing not more than 7 carbon atoms.

6. A process according to any one of claims 1 to 5, wherein a removable group in a compound of the formula II, IIa, IIb or IIc is a methylthio group, a methoxy group or a chlorine or bromine atom.

7. A process according to claim 1, which comprises preparing a novel guanidine derivative of the formula I

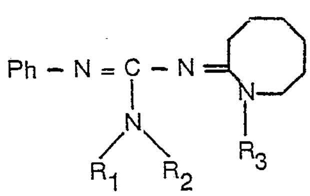

(I)

wherein Ph is a phenyl group which is unsubstituted or substituted by lower alkyl, hydroxy, mercapto, lower alkoxy, lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is lower alkyl or cycloalkyl containing not more than 10 carbon atoms, or both taken together are a bivalent hydrocarbon radical of aliphatic character, which radical is unsubstituted or substituted by lower alkyl or phenyl, and in which radical the carbon atoms of the chain may be interrupted by oxygen, sulfur or nitrogen, and $R_3$ is hydrogen or lower alkyl, or a tautomeric compound or a salt thereof, with the radicals qualified by the term "lower" containing not more than 7 carbon atoms.

8. A process according to claim 1, which comprises preparing a compound of the formula I, wherein Ph is a phenyl group which is unsubstituted or substituted by lower alkyl, hydroxy, mercapto, lower alkoxy,

lower alkenyloxy, lower alkylenedioxy, lower alkylthio, halogen, trifluoromethyl, nitro, amino, lower alkylamino, di-lower alkylamino, sulfamyl, lower alkylsulfamyl, di-lower alkylsulfamyl, carboxy or lower alkoxycarbonyl, each of $R_1$ and $R_2$ independently of the other is a methyl or ethyl, cyclopentyl, cyclohexyl or cycloheptyl radical, or both taken together are a bivalent hydrocarbon radical of aliphatic character containing 4 to 7 carbon atoms in the chain, which radical is unsubstituted or substituted by lower alkyl or phenyl, and in which radical the carbon atoms of the chain may be interrupted by oxygen, sulfur or nitrogen, and $R_3$ is hydrogen or lower alkyl, or a tautomeric compound or a salt thereof, with the radicals qualified by the term "lower" containing not more than 7 carbon atoms.

9. A process according to claim 1, which comprises preparing N-hexahydro-2(1H)-azocinylidene-N'-phenyl-1-pyrrolidinecarboximide-amide.

10. A process according to claim 1, which comprises preparing N-hexahydro-2(1H)-azocinylidene-N'-phenyl-1-piperidinecarboximide-amide.

11. A process according to claim 1, which comprises preparing N-hexahydro-2(1H)-azocinylidene-N'-phenyl-4-morpholinecarboximide-amide.

12. A process according to claim 1, which comprises preparing N-hexahydro-2(1H)-azocinylidene-N'-phenyl-2,6-dimethyl-4-morpholinecarboximid-amide.

**Revendications pour les Etats contractants: BE CH DE FR GB IT LI LU NL SE**

1. Nouveaux dérivés de guanidines de formule I

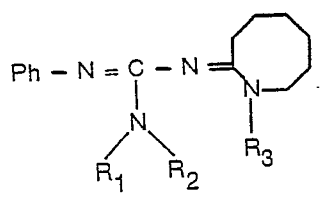

(I)

dans laquelle Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs, des halogènes, des groupes trifluorométhyle, nitro, amino, alkylamino inférieurs, di-(alkyle inférieur)-amino, sulfamyle, alkylsulfamyle inférieurs, di-(alkyle inférieur)-sulfamyle, carboxy ou (alcoxy inférieur)-carbonyle, $R_1$ et $R_2$ représentent chacun, indépendamment l'un de l'autre, un groupe alkyle inférieur ou cycloalkyle contenant au maximum 10 atomes de carbone ou forment ensemble un reste hydrocarboné bivalent à caractère aliphatique éventuellement substitué par des groupes alkyle inférieurs ou phényle et contenant 4 à 7 atomes de carbone dans la chaîne, les atomes de carbone de la chaîne pouvant être interrompus par l'oxygène, le soufre ou l'azote, $R_3$ représente l'hydrogène ou un groupe alkyle inférieur, leurs composés tautomères et leurs sels, les restes qualifiés d'"inférieurs" contenant au maximum 7 atomes de carbone.

2. Composés de formula I dans lesquels Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs, des halogènes, des groupes trifluorométhyle, nitro, amino, alkylamino inférieurs, di-(alkyle inférieur)-amino, sulfamyle, alkylsulfamyle inférieurs, di-(alkyle inférieur)-sulfamyle, carboxy ou (alcoxy inférieur)-carbonyle, $R_1$ et $R_2$ représentent chacun, indépendamment l'un de l'autre, un groupe méthyle ou éthyle, cyclopentyle, cyclohexyle ou cycloheptyle ou forment ensemble un reste hydrocarboné bivalent à caractère aliphatique portant éventuellement des substituants alkyle inférieurs ou phényle et contenant 4 à 7 atomes de carbone dans la chaîne, les atomes de carbone de la chaîne pouvant être interrompus par l'oxygène, le soufre ou l'azote, $R_3$ représente l'hydrogène ou un groupe alkyle inférieur, leurs composés tautomères et leurs sels, les restes qualifiés d'"inférieurs" contenant au maximum 7 atomes de carbone.

3. Composés de formule I dans lesquels Ph représente un reste phényle éventuellement substitué par des groupes alkyle inférieurs, alcoxy inférieurs, des halogènes ou des groupes trifluorométhyle, $R_1$ et $R_2$ forment ensemble une chaîne alkylène inférieur portant éventuellement des substituants alkyle inférieurs ou phényle, les atomes de carbone de la chaîne pouvant être interrompus par l'oxygène, le soufre ou l'azote lui-même éventuellement substitué par des groupes alkyle inférieurs, phényle, benzyle, phényléthyle ou alcoxycarbonyle, et $R_3$ représente l'hydrogène ou un groupe alkyle inférieur, leurs composés tautomères et leurs sels, les restes qualifiés d'"inférieurs" contenant au maximum 7 atomes de carbone.

4. Composés de formule I dans lesquels Ph représente un reste phényle éventuellement substitué par des groupes alkyle inférieurs, alcoxy inférieurs, des halogènes, ou des groupes trifluorométhyle, le groupe $-NR_1R_2$ peut consister en un groupe pyrrolidino, 2,5-diméthylpyrrolidino, pipéridino, 2-méthyl-, 4-méthyl- ou 4-phénylpipéridino, hexahydroazépino, morpholino, 2,6-diméthylmorpholino, 2,6-diméthylmorpholino, thiomorpholino, 2,6-diméthylthiomorpholino, pipérazino, N-méthyl-, N-phényl-, N-benzyl-, N-méthoxy-, N-éthoxy-carbonylpipérazine, et $R_3$ représente l'hydrogène ou un groupe alkyle inférieur, leurs composés tautomères et leurs sels, les restes qualifiés d'"inférieurs" contenant au maximum 7 atomes de carbone.

5. Composés de formule I dans lesquels Ph représente un groupe phényle éventuellement substitué par des groupes alkyle inférieurs, par exemple méthyle ou éthyle, alcoxy inférieurs, par exemple méthoxy ou éthoxy, des halogènes, par exemple le chlore ou le brome, ou des groupes trifluorométhyle, le groupe —$NR_1R_2$ peut consister en un groupe pyrrolidino, pipéridino, 4-méthyl- ou 4-phényl-pipéridino, morpholino, 2,6-diméthylmorpholino, pipérazino, N-méthyl-, N-méthoxy-carbonylpipérazine, et $R_3$ représente l'hydrogène ou un groupe méthyle ou éthyle, leurs composés tautomères et leurs sels.

6. Le N-hexahydro-2(1H)-azocinylidène-N'-phényl-1-pyrrolidino-carboxyimidamide.

7. Le N-hexahydro-2(1H)-azocinylidène-N'-phényl-1-pipéridino-carboximidamide.

8. Le N-hexahydro-2(1H)-azocinylidène-N'-phényl-4-morpholinocarboximidamide.

9. Le N-hexahydro-2(1H)-azocinylidène-4,N'-diphényl-pipéridinocarboximidamide.

10. Le N-hexahydro-2(1H)-azocinylidène-N'-phényl-2,6-diméthyl-4-morpholinocarboximidamide.

11. Le N'-(1-méthyl-hexahydro-2(1H)-azocinylidène)-N-phényl-4-morpholinocarboximidamide.

12. Le N-hexahydro-2(1H)-azocinylidène-N'-(p-fluorophényl)-4-morpholinocarboximidamide.

13. Le N-hexahydro-2(1H)-azocinylidène-N'-(m-trifluorométhylphényl)-4-morpholinocarboximidamide.

14. Le N-hexahydro-2(1H)-azocinylidène-N'-p-tolyl-4-morpholinocarboximidamide.

15. Le N-hexahydro-2(1H)-azocinylidène-N'-phényl-1-(carbéthoxypipérazino)-carboximidamide.

16. Le N-hexahydro-2(1H)-azocinylidène-N'-(p-méthoxyphényl)-1-(4-carbéthoxypipérazino)-carboximidamide.

17. Le N-hexahydro-2(1H)-azocinylidène-N'-phényl-(4-méthylpipérazino)-carboximidamide.

18. Composés tautomères des rev. 6 à 17.

19. Sels des composès des rev. 6 à 18.

20. Sels non toxiques, acceptables pour l'usage pharmaceutique, des composés des rev. 6 à 18.

21. Composés de formule I possédant une activité hypoglycémique et composés tautomères correspondants selon la rev. 1 et sels de ces composés.

22. Compositions pharmaceutiques contenant l'un des composés des rev. 1 à 17 et 20.

23. Les composés des rev. 1 à 17 et 20, pour l'utilisation en tant qu'antidiabétiques.

24. Utilisation des composés des rev. 1 à 17 et 20 pour la préparation de compositions pharmaceutiques.

25. Procédé de préparation des nouveaux dérivés de guanidines de formula I

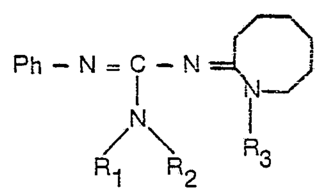

(I)

dans laquelle Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs, des halogènes, des groupes tri-fluorométhyle, nitro, amino, alkylamino inférieurs, di-(alkyle inférieur)-amino, sulfamyle, alkylsulfamyle inférieurs, di-(alkyle inférieur)- sulfamyle, carboxy ou (alcoxy inférieur)-carbonyle, $R_1$ et $R_2$ représentent, indépendamment l'un de l'autre, un groupe alkyle inférieur ou cycloalkyle contenant au maximum 10 atomes de carbone ou forment ensemble un reste hydrocarboné bivalent à caractère aliphatique éventuellement substitué par des groupes alkyle inférieurs ou phényle et contenant 4 à 7 atomes de carbone dans la chaîne, les atomes de carbone de la chaîne pouvant être interrompus par l'oxygène, le soufre ou l'azote, $R_3$ représente l'hydrogène ou un groupe alkyle inférieur, de leurs composés tautomères et de leurs sels, les restes qualifiés d'"inférieurs" contenant au maximum 7 atomes de carbone, caractérisé en ce que:

a) on fait réagir un composé de formule II

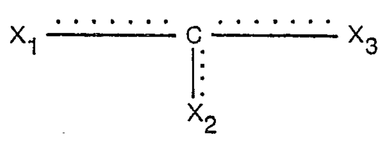

(II)

dans laquelle $X_1$ représente le groupe Ph—N= dans lequel Ph est un groupe phényle éventuellement substitué, ou un groupe éliminable, $X_2$ représente le groupe —$NR_1R_2$ dans lequel $R_1$ et $R_2$ ont les significations indiquées en référence à la formule I, ou un groupe éliminable, et $X_3$ représente le groupe

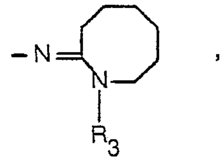

,

dans lequel $R_3$ a les significations indequées en référence à la formule I, ou un groupe éliminable, étant spécifié que l'un seulement des substituants $X_1$, $X_2$ ou $X_3$ peut consister en un groupe éliminable, et l'un des groupes $X_1$, $X_2$ ou $X_3$ est relié avec l'atome de carbone par une double liaison, avec une amine ou une imine correspondant au groupe amino ou imino manquant tel que défini en référence à $X_1$, $X_2$ ou $X_3$, pour remplacement du groupe éliminable, ou bien

b) on fait réagir un composé de formule IV

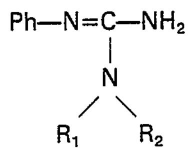

(IV)

dans laquelle Ph, $R_1$ et $R_2$ ont les significations indiquées en référence à la formule I, avec un composé de formule générale V

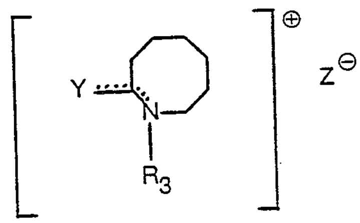

(V) ,

dans laquelle Y représente un groupe alcoxy inférieur, alkylthio inférieur, un halogène ou bien Y représente deux groupes alcoxy inférieurs placés sur le même atome de carbone, et Z représente un anion tétrafluoborate, fluosulfonate, alkylsulfate inférieur ou alcane-sulfonate inférieur ou un halogénure, l'anion Z étant supprimé lorsque Y représente deux groupes alcoxy inférieurs sur le même atome de carbonne, ou bien, lorsque $R_3$ représente l'hydrogène, la forme tautomère est à l'état de base libre, ou bien

c) on convertit des composés de formule I dans laquelle $R_1$ a la signification indiquée ci-dessus et $R_2$ et/ou $R_3$ représentent l'hydrogène, par réaction avec un ester réactif d'un alcanol inférieur ou le cas échéant d'un cycloalcanol, en composés de formule I dans lesquels $R_2$ et/ou $R_3$ ont une signification autre que l'hydrogène dans le cadre des définitions données ci-dessus pour $R_2$ et $R_3$, ou bien

d) dans des composés de formule générale VI

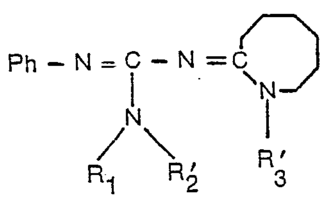

(VI) ,

dans laquelle $R_1$ et Ph ont les significations indiquées en référence à la formule I et l'un des substituants $R_2'$ et $R_3'$ a la signification de $R_2$ ou $R_3$, l'autre étant un groupe protecteur du groupe amino, ou bien $R_2'$ et $R_3'$ représentent tous deux des groupes protecteurs du groupe amino, on éliminé ces groupes et si on le désiré, on exécute des stades opératoires supplémentaires et/ou, si on le désire, on convertit les composés obtenus répondant à la formule I en un sel et/ou, si on le désire, on convertit des sels de composés de formule I ainsi obtenus en les bases libres.

**Revendications pour l'Etat contractant: AT**

1. Procédé de préparation de nouveaux dérivés de guanidines de formule I

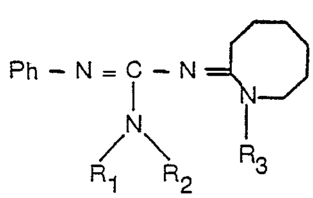

(I)

dans laquelle Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs,

des halogènes, des groupes trifluorométhyle, nitro, amino, alkylamino inférieurs, di-(alkyle inférieur)-amino, sulfamyle, alkylsulfamyle inférieurs, di-(alkyle inférieur)-sulfamyle, carboxy ou (alcoxy inférieur)-carbonyle, $R_1$ et $R_2$ représentent chacun, indépendamment l'un de l'autre, un groupe alkyle inférieur ou cycloalkyle contenant au maximum 10 atomes de carbone ou forment ensemble un reste hydrocarboné bivalent à caractère aliphatique éventuellement substitué par des groupes alkyle inférieurs ou phényle et contenant 4 à 7 atomes de carbone dans la chaîne, les atomes de carbone de la chaîne pouvant être interrompus par l'oxygène, le soufre ou l'azote, $R_3$ représente l'hydrogène ou un groupe alkyle inférieur, leurs composés tautomères et leurs sels, les restes qualifiés d'"inférieurs" contenant au maximum 7 atomes de carbone, caractérisé en ce que:

a) on fait réagir un composé de formule II

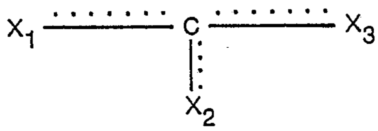

(II)

dans laquelle $X_1$ représente le groupe Ph—N= dans lequel Ph est un groupe phényle éventuellement substitué, ou un groupe éliminable, $X_2$ représente le groupe $—NR_1R_2$ dans lequel $R_1$ et $R_2$ ont les significations indiquées en référence à la formule I, ou un groupe éliminable, et $X_3$ représente le groupe

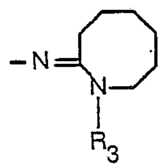

,

dans lequel $R_3$ a les significations indiquées en référence à la formule I, ou un groupe éliminable, étant spécifié que l'un seulement des substituants $X_1$, $X_2$ ou $X_3$ peut consister en un groupe éliminable, et l'un des groupes $X_1$, $X_2$ ou $X_3$ est relié avec l'atome de carbone par une double liaison, avec une amine ou une imine correspondant au groupe amino ou imino manquant tel que défini en référence à $X_1$, $X_2$ ou $X_3$, pour remplacement du groupe éliminable, ou bien

b) on fait réagir un composé de formule IV

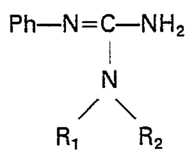

(IV)

dans laquelle Ph, $R_1$ et $R_2$ ont les significations indiquées en référence à la formule I, avec un composé de formule générale V

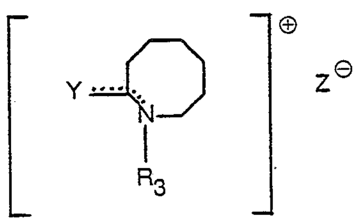

(V) ,

dans laquelle Y représente un groupe alcoxy inférieur, alkylthio inférieur, un halogène ou bien Y représente deux groupes alcoxy inférieurs placés sur le même atome de carbone, et Z représente un anion tétrafluoroborate, fluosulfonate, alkylsulfate inférieur ou alcane-sulfonate inférieur ou un halogénure, l'anion Z étant supprimé lorsque Y représente deux groupes alcoxy inférieurs sur le même atome de carbonne, ou bien, lorsque $R_3$ représente l'hydrogène, la forme tautomère est à l'état de base libre, ou bien

c) on convertit des composés de formule I dans laquelle $R_1$ a la signification indiquée ci-dessus et $R_2$ et/ou $R_3$ représentent l'hydrogène, par réaction avec un ester réactif d'un alcanol inférieur ou le cas échéant d'un cycloalcanol, en composés de formule I dans lesquels $R_2$ et/ou $R_3$ ont une signification autre que l'hydrogène dans le cadre des définitions données ci-dessous pour $R_2$ et $R_3$, ou bien

d) dans des composés de formule générale VI

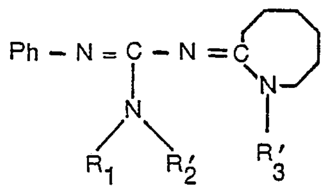

(VI) ,

dans laquelle $R_1$ et Ph ont les significations indiquées en référence à la formule I et l'un des substituants $R'_2$ et $R'_3$ a la signification de $R_2$ ou $R_3$, l'autre étant un groupe protecteur du groupe amino, ou bien $R'_2$ et $R'_3$ représentent tous deux des groupes protecteurs du groupe amino, on élimine ces groupes et si on le désiré, on exécute des stades opératoires supplémentaires et/ou, si on le désire, on convertit les composés obtenus répondant à la formule I en un sel et/ou, si on le désire, on convertit des sels de composés de formule I ainsi obtenus en les bases libres.

2. Procédé selon la rev. 1, caractérisé en ce que l'on fait réagir un composé de formule IIa

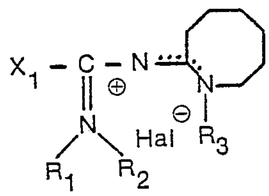

(IIa) ,

dans laquelle $X_1$ représente un groupe éliminable et $R_1$, $R_2$ et $R_3$ ont les significations indiquées en référence à la formule I, avec une aniline éventuellement substituée de formule Ph—$NH_2$.

3. Procédé selon la rev. 1, caractérisé en ce que l'on fait réagir un composé de formule IIb

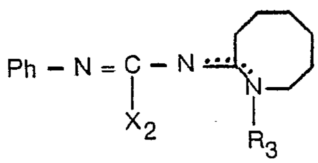

(IIb) ,

dans laquelle $X_2$ représente un groupe éliminable et Ph et $R_3$ ont les significations indiquées en référence à la formule I, avec une amine de formule $HNR_1R_2$.

4. Procédé selon la rev. 1, caractérisé en ce que l'on fait réagir un composé de formule IIc

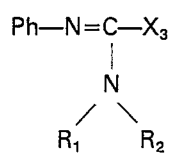

(IIc)

dans laquelle $X_3$ représente un groupe éliminable et Ph, $R_1$ et $R_2$ ont les significations indiquées en référence à la formule I, avec un composé iminé de formule III

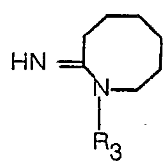

(III)

5. Procédé selon les rev. 1 à 4, caractérisé en ce qu'un reste éliminable des composés de formules II, IIa, IIb et IIc consiste en un groupe alkylthio inférieur, alcoxy inférieur ou un halogène, les restes qualifiés d'"inférieurs" contenant au maximum 7 atomes de carbone.

6. Procédé selon les rev. 1 à 5, caractérisé en ce qu'un reste éliminable des composés de formules II, IIa, IIb, consiste en un groupe méthylthio, méthoxy ou un atome de chlore ou de brome.

7. Procédé selon le rev. 1, caractérisé en ce que l'on prépare de nouveaux dérivés de guanidines de formule I

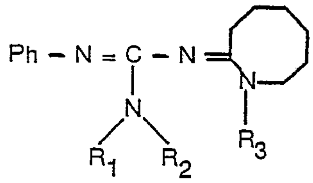

(I)

dans laquelle Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs, des halogènes, des groupes trifluorométhyle, nitro, amino, alkylamino inférieurs, di-(alkyle inférieur-amino, sulfamyle, alkylsulfamyle inférieurs, di-(alkyle inférieur)-sulfamyle, carboxy ou (alcoxy inférieur)-carbonyle, $R_1$ et $R_2$ représentent, indépendamment l'un de l'autre, un groupe alkyle inférieur ou cycloalkyle contenant au maximum 10 atomes de carbone ou forment ensemble un reste hydrocarboné bivalent à caractère aliphatique éventuellement substitué par des groupes alkyle inférieurs ou phényle, les atomes de carbone de la chaîne pouvant être interrompus par l'oxygène, le soufre ou l'azote, $R_3$ représente l'hydrogène ou un groupe alkyle inférieur, leurs composés tautomères et leurs sels, les restes qualifiés d'"inférieurs" contenant au maximum 7 atomes de carbone.

8. Procédé selon la rev. 1, caractérisé en ce que l'on prépare des composés de formule I dans laquelle Ph représente un reste phényle éventuellement substitué par des groupes alkyle inférieurs, hydroxy, mercapto, alcoxy inférieurs, alcényloxy inférieurs, alkylène-dioxy inférieurs, alkylthio inférieurs, des halogènes, des groupes trifluorométhyle, nitro, amino, alkylamino inférieurs, di-(alkyle inférieur)-amino, sulfamyle, alkylsulfamyle inférieurs, di-(alkyle inférieur)-sulfamyle, carboxy ou (alcoxy inférieur)-carbonyle, $R_1$ et $R_2$ représentent chacun, indépendamment l'un de l'autre, un groupe méthyle ou éthyle, cyclopentyle, cyclohexyle ou cycloheptyle ou forment ensemble un reste hydrocarboné bivalent à caractère aliphatique portant éventuellement des substituants alkyle inférieurs ou phényle et contenant 4 à 7 atomes de carbone dans la chaîne, les atomes de carbone de la chaîne pouvant être interrompus par l'oxygène, le soufre ou l'azote, $R_3$ représente l'hydrogène ou un groupe alkyle inférieur, leurs composés tautomères et leurs sels, les restes qualifiés d'"inférieurs" contenant au maximum 7 atomes de carbone.

9. Procédé selon la rev. 1, caractérisé en ce que l'on préparé le N-hexahydro-2(1H)-azocinylidène-N'-phényl-1-pyrrolidino-carboximidamide.

10. Procédé selon la rev. 1, caractérisé en ce que l'on prepare le N-hexahydro-2(1H)-azocinylidène-N'-phényl-1-pipéridinocarboximidamide.

11. Procédé selon la rev. 1, caractérisé en ce que l'on prépare le N-hexahydro-2(1H)-azocinylidène-N'-phényl-4-morpholinocarboximidamide.

12. Procédé selon la rev. 1, caractérisé en ce que l'on prépare le N-hexahydro-2(1H)-azocinylidène-N'-phényl-2,6-diméthyl-4-morpholinocarboximidamide.